# EUROPEAN PATENT SPECIFICATION

(11) **EP 3 649 153 B1**
(45) Date of publication and mention of the grant of the patent: **11.06.2025**
(21) Application number: 18742582.2
(22) Date of filing: 06.07.2018
(51) Int. Cl.: C07K 16/28, C07K 14/55, A61K 39/00

(54) **FC OPTIMISED ANTI-CD25 ANTIBODIES FOR TUMOR SPECIFIC CELL DEPLETION**
FC OPTIMIZIERTE ANTI CD25 ANTIKÖRPER FÜR TUMORSPEZIFISCHEN ZELLABBAU
ANTICOPRS ANTI CD25 OPTIMISÉS DANS LEUR PARTIE FC IMPLIQUANT LA DÉPLÉTION DE CELLULES SPÉCIFIQUES AUX TUMEURS

(30) Priority: 06.07.2017 GB 201710879; 07.09.2017 GB 201714429
(43) Date of publication of application: 13.05.2020
(73) Proprietor: Tusk Therapeutics Ltd, Hertfordshire AL7 1TW (GB); Cancer Research Technology Limited, London EC1V 4AD (GB)
(72) Inventor: MERCHIERS, Pascal, Hertfordshire AL7 1TW (GB); GOUBIER, Anne, Hertfordshire AL7 1TW (GB); MOULDER, Kevin, Hertfordshire AL7 1TW (GB); SALIMU, Josephine, Hertfordshire AL7 1TW (GB); GOYENECHEA CORZO, Beatriz, Hertfordshire AL7 1TW (GB); QUEZADA, Sergio, London Greater London EC1V 4AD (GB); PEGGS, Karl, London Greater London EC1V 4AD (GB); ARCE VARGAS, Frederick, London Greater London EC1V 4AD (GB); SOLOMON, Isabelle, London Greater London EC1V 4AD (GB); MABBITT, Joseph, Hertfordshire AL7 1TW (GB); HOPLEY, Stephanie, Hertfordshire AL7 1TW (GB)
(74) Representative: Callaghan, Dayle Anne
(86) International application number: PCT/GB2018/051923
(87) International publication number: WO 2019/008386

(56) References cited:
- WO-A1-2014/145907
- WO-A2-2005/115451
- TEMMING A ROBIN ET AL: "Cross-reactivity of mouse IgG subclasses to human Fc gamma receptors: Antibody deglycosylation only eliminates IgG2b binding", MOLECULAR IMMUNOLOGY, PERGAMON, GB, vol. 127, 15 September 2020 (2020-09-15), pages 79 - 86, XP086329112, ISSN: 0161-5890, [retrieved on 20200915], DOI: 10.1016/J.MOLIMM.2020.08.015
- BRUHNS PIERRE ET AL: "Specificity and affinity of human Fcgamma receptors and their polymorphic variants for human IgG subclasses", 16 April 2009 (2009-04-16), pages 3716 - 3725, XP093248263, Retrieved from the Internet <URL:https://ashpublications.org/blood/article/113/16/3716/25117/Specificity-and-affinity-of-human-Fc-receptors-and> DOI: 10.1182/blood-
- FREDERICK ARCE VARGAS ET AL: "Fc-Optimized Anti-CD25 Depletes Tumor-Infiltrating Regulatory T Cells and Synergizes with PD-1 Blockade to Eradicate Established Tumors", IMMUNITY., vol. 46, no. 4, April 2017 (2017-04-01), US, pages 577 - 586, XP055487134, ISSN: 1074-7613, DOI: 10.1016/j.immuni.2017.03.013
- JOHN H. SAMPSON ET AL: "A Pilot Study of IL-2R[alpha] Blockade during Lymphopenia Depletes Regulatory T-cells and Correlates with Enhanced Immunity in Patients with Glioblastoma", PLOS ONE, vol. 7, no. 2, 27 February 2012 (2012-02-27), pages e31046, XP055476722, DOI: 10.1371/journal.pone.0031046
- NICOLAS GOUDIN ET AL: "Depletion of Regulatory T Cells Induces High Numbers of Dendritic Cells and Unmasks a Subset of Anti-Tumour CD8+CD11c+ PD-1lo Effector T Cells", PLOS ONE, vol. 11, no. 6, 24 June 2016 (2016-06-24), pages e0157822, XP055393805, DOI: 10.1371/journal.pone.0157822

## Description

### FIELD OF THE INVENTION

The present invention is in the field of cancer immunotherapy and relates to human IgG2 anti-CD25 antibodies and methods of treating cancer, including a method of treating a tumour, wherein the method involves the use of a human IgG2 antibody to CD25.

### BACKGROUND TO THE INVENTION

Cancer immunotherapy involves the use of a subject's own immune system to treat or prevent cancer. Immunotherapies exploit the fact that cancer cells often have subtly different molecules on their surface that can be detected by the immune system. These molecules, or cancer antigens, are most commonly proteins, but also include molecules such as carbohydrates. Immunotherapy thus involves provocation of the immune system into attacking tumour cells via these target antigens. However, malignant tumours, in particular solid tumours, can escape immune surveillance by means of various mechanisms both intrinsic to the tumour cell and mediated by components of the tumour microenvironment. Amongst the latter, tumour infiltration by regulatory T cells (Treg cells or Tregs) and, more specifically, an unfavourable balance of effector T cells (Teff) versus Tregs (i.e. a low ratio of Teff to Treg), have been proposed as critical factors (Smyth M et al., 2014, Immunol Cell Biol. 92, 473-4).

Since their discovery, Tregs have been found to be critical in mediating immune homeostasis and promoting the establishment and maintenance of peripheral tolerance. However, in the context of cancer, their role is more complex. As cancer cells express both self- and tumour-associated antigens, the presence of Tregs, which seek to dampen effector cell responses, can contribute to tumour progression. The infiltration of Tregs in established tumours therefore represents one of the main obstacles to effective anti-tumour responses and to treatment of cancers in general. Suppression mechanisms employed by Tregs are thought to contribute significantly to the limitation or even failure of current therapies, in particular immunotherapies, that rely on induction or potentiation of anti-tumour responses (Onishi H et al, 2012 Anticanc. Res. 32, 997-1003).

Depletion of Tregs as a therapeutic approach for treating cancer is an approach that is supported by studies having shown the contribution of Tregs to tumour establishment and progression in murine models. Moreover, tumour infiltration by Tregs has also been associated with worse prognosis in several human cancers (Shang B et al., 2015, Sci Rep. 5:15179). However, depletion of Tregs in tumours is complex, and results of studies in this area have been discrepant.

Among the potential molecular targets for achieving depletion of Tregs, the IL-2/CD25 interaction has been the object of several studies in murine models, most of them involving the use of PC61, a rat anti-murine CD25 mouse antibody (Setiady Y et al., 2010. Eur J Immunol. 40:780-6), The CD25 binding and functional activities of this antibody have been compared to those of panel of monoclonal antibodies generated by different authors (Lowenthal J.W et al., 1985. J. Immunol., 135, 3988-3994; Moreau, J.-L et al., 1987. Eur. J. Immunol. 17,929-935; Volk HD et al., 1989 Clin. exp. Immunol. 76, 121-5; Dantal J et al., 1991, Transplantation 52:110-5).

Some available anti-CD25 antibodies, such as PC61, block or inhibit the binding of IL-2 to CD25, as do many other anti-mouse CD25 antibodies (and most of those disclosed as being anti-human CD25 antibodies; see for instance WO2004/045512, WO 2006/108670, WO1993/011238, and WO1990/007861). For example, basiliximab and daclizumab are IgG1 anti-human CD25 antibodies that inhibit the binding of IL-2 to CD25 and have been developed to reduce activation of T-effector cells. Basiliximab is a chimeric mouse-human CD25 antibody currently approved for graft versus host diseases and daclizumab is a humanised CD25 antibody approved for the treatment of multiple sclerosis. Furthermore, other anti-CD25 antibodies still allow the binding of IL-2 to CD25, such as 7D4 (anti-mouse CD25) or clone M-A251 (anti-human CD25) or 7G7B6 (anti-human CD25). Moreover, the binding of anti-CD25 can be characterised with respect to CD25 posttranslational modifications. For instance, ADP-ribosylation of mouse CD25 does not affect the binding of PC61 but it affects the binding of 7D4 to this antigen (Teege S et al., 2015, Sci Rep 5: 8959).

Some literature refers to the use of anti-CD25, alone or in combination, in cancer or in connection to Treg depletion. (WO2004/074437; WO2006/108670; WO2006/050172; WO2011/077245; WO2016/021720; WO2004/045512; Grauer O et al., 2007 Int. J. Cancer: 121: 95-105). However, when tested in mouse models of cancer, the rat anti-mouse CD25 PC61 failed to demonstrate anti-tumour activity when delivered after tumour establishment.

In the context of a murine model of autoimmunity, the anti-CD25 PC61 antibody was re-engineered to evaluate the effect of a highly divergent Fc effector function within an anti-CD25 antibody on IL-2 receptor blocking and depletion of peripheral Treg (Huss D et al., 2016. Immunol. 148: 276-86). However, the ability to deplete Tregs in tumours, or to mediate anti-tumour therapeutic activity, had never been evaluated for PC61 (as such, as an engineered antibody, or as an anti-human CD25 designed or characterized as having CD25 binding features similar to those of PC61 for mouse CD25), in all human IgG isotypes. Furthermore, daclizumab has been engineered as a human IgG2M3 variant to lengthen the antibodies half-life (WO2005/123780). However, this IgG2M3 variant is a specific Fc-silent variant of IgG2 that does not bind any Fc receptors but does bind neonatal Fc receptor (FcRn). Therefore, this IgG2M3 variant is not able to deplete CD25+ cells or deplete Treg cells, via ADCC or ADCP.

7D4 is a rat IgM anti-mouse CD25 antibody that has been extensively used in research to detect CD25-positive cells in the presence of or following the treatment with PC61 or of antibodies having similar binding properties (Onizuka S et al., 1999. Canc Res. 59, 3128 - 3133). FREDERICK ARCE VARGAS ET AL: "Fc-Optimized Anti-CD25 Depletes Tumor-Infiltrating Regulatory T Cells and Synergizes with PD-1 Blockade to Eradicate Established Tumors", IMMUNITY., vol. 46, no. 4, April 2017 (2017-04), pages 577-586, demonstrates that CD25 expression is largely restricted to tumor-infiltrating Treg cells in mice and humans. Existing anti-CD25 antibodies were observed to deplete Treg cells in the periphery. Upregulation of the inhibitory Fc gamma receptor (FcγR) IIb at the tumor site prevented intra-tumoral Treg cell depletion, which may underlie the lack of anti-tumor activity previously observed in pre-clinical models. Use of an anti CD25 antibody with enhanced binding to activating FcγRs led to effective depletion of tumor-infiltrating Treg cells, increased effector to Treg cell ratios, and improved control of established tumors. Combination with anti-programmed cell death protein-1 antibodies promoted complete tumor rejection.

Thus, there is a need in the art for further anti-CD25 antibodies involved in depletion of CD25+ cells, in particular Tregs for treating cancer.

### SUMMARY OF THE INVENTION

The present invention provides novel anti-CD25 antibodies and novel uses of these anti-CD25 antibodies that are characterized by structural elements that allow efficient depletion of CD25+ cells, in particular Tregs, in particular, within tumours.

The inventors found that surprisingly, and contrary to the prevailing wisdom in the field, anti-CD25 antibodies of the human IgG2 isotype promoted efficacious depletion of tumour infiltrating Treg cells and increased the CD4+eff/Treg and CD8+/Treg ratios.

In a main aspect, the present invention provides a human IgG2 anti-CD25 antibody that depletes CD25+ cells, in particular tumour-infiltrating regulatory T cells. The invention also provides a method of treating a human subject who has cancer comprising the step of administering a human IgG2 anti-CD25 antibody to a subject, wherein said subject has a tumour (preferably a solid tumour or a liquid tumour). The anti-CD25 antibody is a human IgG2 antibody, that may be either a blocking or a non-blocking antibody with respect to the binding of CD25 to IL-2, and preferably depletes tumour-infiltrating regulatory T cells. The antibodies of and used in the invention may bind to FcγRIIa.

Antibodies of and used in the invention preferably have a dissociation constant (K_{d}) for CD25 of less than 10⁻⁸ M and/or a dissociation constant for at least one activating Fcγ receptor of less than about 10⁻⁶ M. Most preferably, the human IgG2 anti-CD25 is characterised by other features related to Fcγ receptors, in particular:
(a) binds to Fcγ receptors with an activatory to inhibitory ratio (A/I) superior to 1; and/or
(b) binds to FcγRIIa with higher affinity than it binds to FcγRIIb.

Since the anti-CD25 IgG2 antibodies of the invention deplete CD25+ cells and in particular deplete tumour-infiltrating regulatory T cells, the skilled person would appreciate the human IgG2 antibodies of and used in the invention, are not of the human IgG2M3 variant isotype. An IgG2M3 isotype may be prepared by replacing IgG1 Fc portion of an IgG1 antibody with an IgG2a Fc domain with replacements of alanine for valine at positions 234 and 237 to render the antibody Fc domain incapable of binding FcR and leaving FcRn binding ability intact (Zhang et al., 2014, Mult Scler., 20(2):156-164). IgG2M3 antibodies may therefore be referred to as IgG2a antibodies with replacements of alanine for valine at positions 234 and 237.

In one aspect, the human IgG2 antibody inhibits the binding of interleukin-2 (IL-2) to CD25 (i.e. a blocking antibody). These antibodies are characterised by structural elements that allow binding to CD25 whilst blocking the binding of IL-2 to CD25. Alternatively, the human IgG2 antibody does not inhibit interleukin-2 signalling in CD25-expressing cells, preferably the antibody does not inhibit the binding of interleukin-2 (IL-2) to CD25 (i.e. a non-blocking antibody). Such "non-blocking" human IgG2 anti-CD25 antibodies are characterised by structural elements that allow both binding CD25 without blocking the signalling (and optionally the binding) of interleukin 2 to CD25.

References to "non-blocking" herein (with respect to the non-blocking of IL-2 signalling or binding to CD25 in the presence of the anti-CD25 antibody) include embodiments wherein the anti-CD25 antibody of the invention inhibit less than 50% of IL-2 signalling compared to IL-2 signalling in the absence of the antibodies. Preferably the anti-CD25 antibody inhibits less than about 40%, 35%, 30%, preferably less than about 25% of IL-2 signalling compared to IL-2 signalling in the absence of the antibodies.

Given the use of the anti-CD25 antibody in therapeutic methods, it can further present further preferred features. The anti-CD25 antibody is preferably a monoclonal antibody, in particular a human, chimeric, or humanised antibody. The human IgG2 anti-CD25 antibody may elicit a CDC, ADCC and/or ADCP response, preferably an ADCC and/or ADCP response, preferably an ADCP response. Moreover, in view of its interactions with immune cells and/or other components of the immune system for exerting its activities, the human IgG2 anti-CD25 antibody may further elicit an enhanced CDC, ADCC and/or ADCP response, preferably an increased ADCC and/or ADCP response, more preferably an increased ADCC response. The antibodies may be engineered to enhance the CDC/ADCC and ADCP response as compared to a parental antibody.

In a further aspect the present invention provides a monoclonal human IgG2 anti-CD25 antibody which depletes CD25+ cells, in particular tumour-infiltrating regulatory T cells. Preferably the human IgG2 anti-CD25 antibody elicits ADCP activity.

The human IgG2 anti-CD25 antibody of the present invention (as generally defined above and in further details in the Detailed Description) can be used in methods of treating a human subject wherein said human IgG2 anti-CD25 antibody is administered to a subject who has cancer. In one embodiment the subject has an established, solid tumour or a liquid tumour (optionally in a method further comprising the step of identifying a subject who has a solid tumour or a liquid tumour). Such methods may further comprise administering a further therapeutic agent, in particular an immune checkpoint inhibitor to said subject, for example in the form of an antibody binding and inhibiting an immune checkpoint protein. A preferred immune checkpoint inhibitor is a PD-1 antagonist, which can be an anti-PD-1 antibody or an anti-PD-L1 antibody.

In general, a human IgG2 anti-CD25 antibody of the invention can be used in methods of depleting CD25+ cells, in a subject having cancer, comprising the step of administering said human IgG2 anti-CD25 antibody to said subject. More in general, a human IgG2 anti-CD25 antibody of the invention can be used in methods of depleting regulatory T cells in a subject having cancer comprising the step of administering said human IgG2 anti-CD25 antibody to said subject. Preferably the human IgG2 anti-CD25 antibodies of the invention can be used in methods of depleting regulatory T cells in a solid tumour of a subject having cancer or in methods of depleting regulatory T cells in a liquid tumour of a subject having cancer.

In a further aspect, the human IgG2 anti-CD25 antibody of the invention can be used for the manufacture of a medicament for the treatment of cancer in a human subject, preferably wherein said subject has a solid tumour or a liquid tumour. At this scope, said antibody is for administration in combination with a further therapeutic agent, for example with an immune checkpoint inhibitor, preferably a PD-1 antagonist.

In a further aspect, the present invention provides a combination of a human IgG2 anti-CD25 antibody as defined above with another anti-cancer compound (preferably an immune checkpoint inhibitor or other compounds as indicated in the Detailed Description) for use in the treatment of cancer in a human subject, preferably wherein said subject has a solid tumour or a liquid tumour. The anti-cancer compound (for example, an immune checkpoint inhibitor such a PD-1 antagonist or a cytokine such as Interleukin 2) can be administered simultaneously, separately or sequentially. At this scope the present invention also provides a kit for use in the treatment of cancer comprising a human IgG2 anti-CD25 antibody, as defined above, and an anti-cancer compound (for example, an immune checkpoint inhibitor such as a PD-1 antagonist).

In a further aspect, the present invention also provides a pharmaceutical composition comprising a human IgG2 anti-CD25 antibody as defined above in a pharmaceutically acceptable medium. Such composition may also comprise an anti-cancer compound (for example, an immune checkpoint inhibitor such as a PD-1 antagonist).

The antibodies of the invention may be provided as an injectable pharmaceutical composition. In one aspect, the present invention provides an injectable pharmaceutical composition comprising a human IgG2 anti-CD25 antibody as defined above, and a pharmaceutically acceptable carrier. A further aspect of the invention provides an injectable pharmaceutical composition comprising a monoclonal human IgG2 anti-CD25 antibody of the invention that depletes CD25+ cells, in particular tumour-infiltrating regulatory T cells. The human IgG2 anti-CD25 antibody can preferably elicit an ADCP response. The anti IgG2 anti-CD25 antibody may also be humanised.

In one embodiment, there is provided an injectable pharmaceutical composition comprising a monoclonal human IgG2 anti-CD25 antibody of the invention that depletes CD25+ cells, in particular tumour-infiltrating regulatory T cells and a pharmaceutically acceptable carrier for use in the treatment of cancer. There is also provided a method of treating cancer comprising administering an injectable pharmaceutical composition comprising a monoclonal human IgG2 anti-CD25 antibody of the invention that depletes CD25+ cells, in particular tumour-infiltrating regulatory T cells and a pharmaceutically acceptable carrier. The anti IgG2 anti-CD25 antibody may also be humanised. The human IgG2 anti-CD25 antibody preferably elicit an ADCP response. The antibody may be present in the injectable pharmaceutical formulations at a concentration of 1mg/mL to 50 mg/ml. Preferably the antibody may be present in the injectable pharmaceutical formulations at a concentration of at least 20 mg/ml.

In a further embodiment, there is provided an injectable pharmaceutical composition comprising a monoclonal human IgG2 anti-CD25 antibody of the invention that depletes CD25+ cells, in particular tumour-infiltrating regulatory T cells and a pharmaceutically acceptable carrier for use in the treatment of a solid tumour. There is also provided a method of treating a solid tumour comprising administering an injectable pharmaceutical composition comprising a monoclonal human IgG2 anti-CD25 antibody of the invention that depletes tumour-infiltrating regulatory T cells and a pharmaceutically acceptable carrier. The human IgG2 anti-CD25 antibody may also be humanised. The human IgG2 anti-CD25 antibody preferably elicit an ADCP response. The antibody may be present in the injectable pharmaceutical formulations at a concentration of 1mg/mL to 50 mg/ml. The antibody may be present in the injectable pharmaceutical formulations at a concentration of at least 20 mg/ml.

In a still further aspect, the present invention also provides a bispecific antibody comprising:
(a) a first antigen binding moiety that binds to CD25; and
(b) a second antigen binding moiety that binds to an immune checkpoint protein, a tumour-associated antigen, an anti-human activatory Fc Receptor antibody (FcgRI, FcgRlla, FcgRIII), or an antagonistic anti-human FcγRIIb antibody;
wherein the bispecific antibody is a human IgG2 antibody that depletes CD25+ cells, in particular depletes tumour-infiltrating regulatory T cells. The bispecific antibodies may be provided in an injectable pharmaceutical composition, for example as described above.

Preferably the bispecific antibody is a human IgG2 antibody that depletes tumour-infiltrating regulatory T cells. Preferably the human IgG2 antibody may bind to the activatory Fcγ receptor FcγRIIa.

In one aspect, the CD25 binding moiety inhibits the binding of interleukin-2 to CD25. Alternatively, the CD25 binding moiety does not inhibit the binding of interleukin-2 to CD25.

Preferably, such bispecific antibody comprises a second antigen binding moiety that binds an immune checkpoint protein that is selected from the group consisting of PD-1, CTLA-4, BTLA, KIR, LAG3, VISTA, TIGIT, TIM3, PD-L1, B7H3, B7H4, PD-L2, CD80, CD86, HVEM, LLT1, GAL9, GITR, OX40, CD137, and ICOS. Such immune checkpoint protein is preferably expressed on a tumour cell, and most preferably is selected from PD-1, PD-L1, and CTLA-4. The second antigen binding moiety that binds to an immune checkpoint protein can be comprised in a commercially available antibody that acts as an immune checkpoint inhibitor, for example:
(a) in the case of PD-1, the anti-PD-1 antibody can be nivolumab or pembrolizumab.
(b) In the case of PD-L1, the anti-PD-L1 can be atezolizumab;
(c) In case of CTLA-4, the anti-CTLA-4 can be ipilimumab.

Such bispecific antibodies can be provided in any commercially available format, including Duobody, BiTE DART, CrossMab, Knobs-in-holes, Triomab, or other appropriate molecular format of bispecific antibody and fragments thereof.

Alternatively, such bispecific antibody comprises a second antigen binding moiety that binds to tumour-associated antigen. In this alternative embodiment, such antigens and corresponding antibodies include, without limitation CD22 (blinatumomab), CD20 (rituximab, tositumomab), CD56 (lorvotuzumab), CD66e/CEA (labetuzumab), CD152/CTLA-4 (ipilimumab), CD221/IGF1R (MK-0646), CD326/Epcam (edrecolomab), CD340/HER2 (trastuzumab, pertuzumab), and EGFR (cetuximab, panitumumab).

The combination of human IgG2 anti-CD25 antibody of the invention with another anti-cancer compound, as well as the bispecific antibodies as defined above, can be used in a method of treating cancer, comprising the step of administering said combination or said bispecific antibody to a subject, in particular when the subject has a solid tumour or a liquid tumour, and for use in the treatment of cancer in a subject.

Further objects of the invention, including further definitions of the anti-human CD25 antibodies of the invention and of their uses in methods for treating cancer, in pharmaceutical compositions, in combinations with other anti-cancer compounds, in bispecific antibodies, are provided in the Detailed Description and in the Examples.

### DETAILED DESCRIPTION OF THE INVENTION

The present invention provides a human IgG2 anti-CD25 antibody. The invention also provides a method of treating or preventing cancer, in particular a solid tumour or a liquid tumour, in a subject, comprising the step of administering a human IgG2 antibody that binds to CD25 to said subject, whereby the human IgG2 anti-CD25 antibodies are characterised by structural elements that allow depleting efficiently CD25+ cells, in particular Tregs, in particular within tumours. The human IgG2 antibody that binds to CD25 as defined in the present invention can be used in the treatment or prevention of cancer, in particular, a solid tumour or a liquid tumour. Alternatively put, the present invention provides the use of a human IgG2 antibody that binds to CD25 and allows efficient depletion of CD25+ cells, in particular Tregs for the manufacture of a medicament for the treatment or prevention of cancer, in particular, a solid tumour or a liquid tumour. The invention also provides the use of a human IgG2 antibody that binds CD25 and that allows depletion of CD25+ cells, in particular the depletion of Tregs in the treatment or prevention of cancer, in particular a solid tumour or a liquid tumour.

In one aspect the anti-CD25 antibody, may be a human IgG2 that binds to CD25 and allows both interleukin 2 binding to CD25 and that allows efficient depletion of CD25+ cells, in particular Tregs. Alternatively, the anti-CD25 antibody may be a human IgG2 that allows binding to CD25 without interfering with interleukin 2 binding to CD25 and that allows efficient depletion of CD25+ cells, in particular Tregs.

The present invention discloses how switching the isotype of an anti-CD25 antibody (exemplified by the rat anti-mouse CD25 antibody PC61) to human IgG2 isotype, not known to be a depleting isotype, surprisingly leads to depletion of regulatory T cells.

Moreover, the present inventors have found that CD25 can be targeted using human IgG2 anti-CD25 antibody for depletion of CD25+ cells, in particular regulatory T cells in the therapeutic context, for example in an established solid tumour or in a liquid tumour. The present inventors have found that an anti-CD25 antibody engineered to the human IgG2 isotype leads to effective depletion of tumour-infiltrating regulatory T cells, a therapeutic approach that could, for example, be associated (in combination with or within bispecific antibodies) with other cancer-targeting compounds, such as those targeting an immune checkpoint protein, a tumour-associated antigen, or an inhibitory Fcγ receptor.

CD25 is the alpha chain of the IL-2 receptor, and is found on activated T cells, regulatory T cells, activated B cells, some thymocytes, myeloid precursors and oligodendrocytes. CD25 associates with CD122 and CD132 to form a heterotrimeric complex that acts as the high-affinity receptor for IL-2. The consensus sequence of human CD25 is shown below in SEQ ID NO: 1 (Uniprot accession number P01589; the extracellular domain of mature human CD25, corresponding to amino acids 22-240, is underlined and is presented as SEQ ID NO: 2):

10 20 30 40 50
MDSYLLMWGL LTFIMVPGCQ AELCDDDPPE IPHATFKAMA YKEGTMLNCE
60 70 80 90 100
CKRGFRRIKS GSLYMLCTGN SSHSSWDNQC QCTSSATRNT TKQVTPQPEE
110 120 130 140 150
QKERKTTEMQ SPMQPVDQAS LPGHCREPPP WENEATERIY HFVVGQMVYY
160 170 180 190 200
QCVQGYRALH RGPAESVCKM THGKTRWTQP QLICTGEMET SQFPGEEKPQ
210 220 230 240 250
ASPEGRPESE TSCLVTTTDF QIQTEMAATM ETSIFTTEYQ VAVAGCVFLL
260 270
ISVLLLSGLT WQRRQRKSRR TI

As used herein, "an antibody that binds CD25" refers to an antibody that is capable of binding to the CD25 subunit of the IL-2 receptor. This subunit is also known as the alpha subunit of the IL-2 receptor. Such an antibody is also referred to herein as an "anti-CD25 antibody".

An anti-CD25 antibody is an antibody capable of specific binding to the CD25 subunit (antigen) of the IL-2 receptor. "Specific binding", "bind specifically", and "specifically bind" are understood to mean that the antibody has a dissociation constant (K_{d}) for the antigen of interest of less than about 10⁻⁶ M, 10⁻⁷ M, 10⁻⁸ M, 10⁻⁹ M, 10⁻¹⁰ M, 10⁻¹¹ M or 10⁻¹² M. In a preferred embodiment, the dissociation constant is less than 10⁻⁸ M, for instance less than 10⁻⁹ M, 10⁻¹⁰ M, 10⁻¹¹ M or 10⁻¹² M.

The Kd to evaluate the binding affinity of the human IgG2 antibody to CD25 can be obtained by standard methodologies, including surface plasmon resonance (SPR), such as by Biacore analysis, or analysis using Forte Bio Octet Systems, and as described in the Examples.

For example, measuring K_{D} by biolayer interferometry, may involve using the Octet Red 96 system (Pall Forte Bio Corp., USA). Sensors are equilibrated off-line in kinetic buffer for 10 minutes and then monitored on-line for 60 seconds for baseline establishment. 0.625ug/ml of recombinant human CD25 his tagged is loaded onto Ni-NTA Biosensors for 300s. After wash for 15 seconds and a base line step for 45 seconds on kinetic buffer, sensors are exposed to a series of antibody concentrations (33.3, 8.3, 2.2 and 0.5 nM) of antibody for 1800 seconds followed by dissociation in kinetics buffer for 1200 seconds. Data is processed using Forte Bio Data Analysis Software 9.0, with reference subtraction.

As used herein, the term "antibody" refers to both intact immunoglobulin molecules as well as fragments thereof that include the antigen-binding site, and includes polyclonal, monoclonal, genetically engineered and otherwise modified forms of antibodies, including but not limited to chimeric antibodies, humanised antibodies, heteroconjugate and/or multispecific antibodies (e.g., bispecific antibodies, diabodies, tribodies, and tetrabodies), and antigen binding fragments of antibodies, including e.g. Fab', F(ab')₂, Fab, Fv, rIgG, polypeptide-Fc fusions, single chain variants (scFv fragments, VHHs, Trans-bodies^{®}, Affibodies^{®}, shark single domain antibodies, single chain or Tandem diabodies (TandAb^{®}), VHHs, Anticalins^{®}, Nanobodies^{®}, minibodies, BiTE^{®}s, bicyclic peptides and other alternative immunoglobulin protein scaffolds). In some embodiments, an antibody may lack a covalent modification (e.g., attachment of a glycan) that it would have if produced naturally. In some embodiments, an antibody may contain a covalent modification (e.g., attachment of a glycan, a detectable moiety, a therapeutic moiety, a catalytic moiety, or other chemical group providing improved stability or administration of the antibody, such as poly-ethylene glycol). In some embodiments, the antibody may be in the form of a masked antibody (e.g. Probodies ^{®}). A masked antibody can comprise a blocking or "mask" peptide that specifically binds to the antigen binding surface of the antibody and interferes with the antibody's antigen binding. The mask peptide is linked to the antibody by a cleavable linker (e.g. by a protease). Selective cleavage of the linker in the desired environment, i.e. in the tumour environment, allows the masking/blocking peptide to dissociate, enabling antigen binding to occur in the tumour, and thereby limiting potential toxicity issues.

"Antibody" may also refer to camelid antibodies (heavy-chain only antibodies) and antibody-like molecules such as anticalins (Skerra (2008) FEBS J 275, 2677-83). In some embodiments, an antibody is polyclonal or oligoclonal, that is generated as a panel of antibodies, each associated to a single antibody sequence and binding more or less distinct epitopes within an antigen (such as different epitopes within human CD25 extracellular domain that are associated to different reference anti-human CD25 antibodies). Polyclonal or oligoclonal antibodies can be provided in a single preparation for medical uses as described in the literature (Kearns JD et al., 2015. Mol Cancer Ther. 14:1625-36).

In one aspect of the invention the antibody is monoclonal. The antibody may additionally or alternatively be humanised or human. In a further aspect, the antibody is human, or in any case an antibody that has a format and features allowing its use and administration in human subjects.

Antibodies (Abs) and immunoglobulins (Igs) are glycoproteins having the same structural characteristics. Immunoglobulins may be from any class such as IgA, IgD, IgG, IgE or IgM. Immunoglobulins can be of any subclass such as IgG₁, IgG₂, IgG₃, or IgG₄. In the present invention the anti-CD25 antibody is from the human IgG₂ subclass.

The Fc region of IgG antibodies interacts with several cellular Fcγ receptors (FcγR) to stimulate and regulate downstream effector mechanisms. There are five activating receptors, namely FcγRI (CD64), FcγRIIa (CD32a), FcγRIIc (CD32c), FcγRIIIa (CD16a) and FcγRIIIb (CD16b), and one inhibitory receptor FcγRIIb (CD32b). The communication of IgG antibodies with the immune system is controlled and mediated by FcγRs, which relay the information sensed and gathered by antibodies to the immune system, providing a link between the innate and adaptive immune systems, and particularly in the context of biotherapeutics (Hayes J et al., 2016. J Inflamm Res 9: 209-219).

IgG subclasses vary in their ability to bind to FcγR and this differential binding determines their ability to elicit a range of functional responses. For example, in humans, FcγRIIIa is the major receptor involved in the activation of antibody-dependent cell-mediated cytotoxicity (ADCC) and IgG3 followed closely by IgG1 display the highest affinities for this receptor, reflecting their ability to potently induce ADCC. Whilst IgG2 have been shown to have weak binding for this receptor. Despite this weak binding the inventors have found that anti-CD25 having the human IgG2 isotype efficiently deplete Tregs.

In a particular embodiment of the invention, the antibody binds the activating receptor FcγRIIa. In a particular embodiment, the antibody binds to the activatory FcγIIa receptor with a dissociation constant of less than about 10⁻⁶ M, 10⁻⁷ M, 10⁻⁸ M, 10⁻⁹ M or 10⁻¹⁰ M. In a particular embodiment, the antibody binds to at least one activatory Fcγ receptor with a dissociation constant of less than about 10⁻⁶ M.

In one aspect, the antibody binds an inhibitory receptor, FcγRIIb, with low affinity. In one aspect, the antibody binds FcγRIIb with a dissociation constant higher than about 10⁻⁷ M, higher than about 10⁻⁶ M or higher than about 10⁻⁵ M. In a particular embodiment, the antibody binds FcγRIIb with a dissociation constant higher than about 10⁻⁷ M. In a particular embodiment the antibody does not bind the inhibitory Fc receptor, FcγRIIb.

The Kd to evaluate the binding affinity of the human IgG2 antibody to Fcγ receptors can be obtained by standard methodologies, including analysis using Forte Bio Octet Systems, and as described in the Examples.

In a preferred embodiment of the invention, the human IgG2 anti-CD25 antibody has ADCC and/or ADCP activity, as discussed herein, in particular with respect to cells of human origin. As previously described (Nimmerjahn F et al., 2005. Science, 310:1510-2), the mIgG2a isotype (which corresponds human IgG1 isotype) binds to all FcγR subtypes with a high activatory to inhibitory ratio (A/I), that is at least superior to 1. In contrast, other isotypes (such as rIgG1 isotype) bind with a similar affinity to a single activatory FcγR only (FcγRIII), as well as the inhibitory FcγRIIb, resulting in a low A/I ratio (<1). This lower A/I ratio can correlate with a lower in intra-tumoral Treg depletion and lower anti-tumour therapeutic activity of the isotype. However, the inventors have surprisingly found that despite the known FcγR binding profile for antibodies of the human IgG2 isotype, significant Treg depletion was achieved with a human IgG2 isotype of an anti-CD25 antibody.

In a preferred embodiment, the human IgG2 anti-CD25 antibody as described herein binds human CD25, preferably with high affinity. Still preferably, the human IgG2 anti-CD25 antibody binds to extracellular region of human CD25, as shown above. In one aspect, the invention provides an anti-CD25 antibody as described herein. In particular, the Examples provide experimental data generated with the human IgG2 antibodies based on those secreted by PC-61.5.3 hybridoma and that generally identified as either PC61 or PC-61. The assays involving PC-61 and mouse CD25 in the literature (for example Setiady Y et al., 2010. Eur. J. Immunol. 40: 780-6; McNeill A et al., 2007. Scand J Immunol. 65:63-9; Teege S et al., 2015, Sci Rep 5: 8959), can be adapted for characterising those human antibodies that recognize human CD25 having the same functional features of PC61 both at the level of interaction with CD25 (in particular, by blocking IL-2 binding) and with Fcγ receptors (in particular by preferably binding human activating Fcγ receptors and depleting efficiently Tregs). Suitable methods will be known to one skilled in the art to achieve the required functional features of the antibody as described herein.

Human IgG2 antibodies can also be developed based on the antibody that is secreted by the 7D4 hybridoma or the non-IL-2 blocking anti-human CD25 antibody named M-A251. As indicated in the Background of the Invention, 7D4 is specific for mouse CD25 which binds to one epitope within mouse CD25 that is distinct from the IL-2 binding site and does not block binding of IL-2 to CD25. The assays involving 7D4 and mouse CD25 in the literature (for example Setiady Y et al., 2010. Eur. J. Immunol. 40: 780-6; McNeill A et al.,2007. Scand J Immunol. 65:63-9; Teege S et al., 2015, Sci Rep 5: 8959), can be adapted for characterising those human antibodies that recognize human CD25 having the same functional features of 7D4 both at the level of interaction with CD25 (in particular, by not blocking IL-2 binding) and with Fcγ receptors (in particular by preferably binding one or more of the human activating Fcγ receptors and depleting efficiently CD25+ cells, in particular depleting Treg),

In a preferred embodiment, the method of treating a human subject who has a cancer comprises the step of administering a human IgG2 anti-CD25 antibody of the invention to a subject, wherein said subject preferably has a solid tumour or a liquid tumour, and wherein the human IgG2 anti-CD25 antibody depletes CD25+ cells, in particular tumour-infiltrating regulatory T cells. In one particular embodiment the human IgG2 anti-CD25 antibody binds the activating Fcγ receptor FcγRIIa (CD32a). Preferably the anti-CD25 antibody has a dissociation constant (K_{d}) for CD25 of less than 10⁻⁸ M. More preferably, the anti-CD25 antibody binds human CD25 providing effects on IL-2 binding and Treg depletion similar to those on mouse CD25. In a further embodiment, the anti-CD25 antibody binds to Fcγ receptors with an activatory to inhibitory ratio (A/I) superior to 1 and/or binds to FcγRIIa (CD32a) with higher affinity than it binds to FcγRIIb (CD32b).

The CD25 binding domain of PC-61 antibody has been cloned and expressed as a recombinant protein in fusion with an appropriate constant region. The sequence of the CD25 binding domain of PC-61 antibody, as well its specificity for distinct epitopes within the extracellular domain of CD25 and/or its other functional activities, can be used for comparing candidate anti-CD25 antibodies that are generated and screened by any appropriate technique (e.g. by raising panels of hybridomas from CD25-immunized rodents or generating libraries of recombinant antibodies and then screening these antibody repertoires with CD25 fragments for characterising functionally as described herein). The anti-CD25 antibodies that are consequently identified can be produced also as recombinant antibodies, in particular as full antibodies or as fragments or variants that are described herein.

Native antibodies and immunoglobulins are usually heterotetrameric glycoproteins of about 150,000 daltons, composed of two identical light (L) chains and two identical heavy (H) chains. Each heavy chain has at the amino terminus a variable domain (V_{H}) followed by a number of constant domains. Each light chain has a variable domain at the amino terminus (V_{L}) and a constant domain at the carboxy terminus.

The variable regions are capable of interacting with a structurally complementary antigenic target and are characterized by differences in amino acid sequence from antibodies of different antigenic specificity. The variable regions of either H or L chains contain the amino acid sequences capable of specifically binding to antigenic targets. Within these sequences are smaller sequences dubbed "hypervariable" because of their extreme variability between antibodies of differing specificity. Such hypervariable regions are also referred to as "complementarity determining regions" or "CDR" regions.

These CDR regions account for the basic specificity of the antibody for a particular antigenic determinant structure. The CDRs represent non-contiguous stretches of amino acids within the variable regions but, regardless of species, the positional locations of these critical amino acid sequences within the variable heavy and light chain regions have been found to have similar locations within the amino acid sequences of the variable chains. The variable heavy and light chains of all antibodies each have 3 CDR regions, each non-contiguous with the others (termed L1, L2, L3, H1, H2, H3) for the respective light (L) and heavy (H) chains. The accepted CDR regions have been described previously (Kabat et al., 1977. J Biol Chem 252, 6609-6616).

The antibodies of the present invention may function through complement-dependent cytotoxicity (CDC) and/or antibody-dependent cell-mediated cytotoxicity (ADCC) and/or antibody-dependent cell-mediated phagocytosis (ADCP), as well as any other mechanism that allows targeting, blocking proliferation, and/or depleting CD25+ cells, in particular Treg cells.

"Complement-dependent cytotoxicity" (CDC) refers to lysis of antigen-expressing cells by an antibody of the invention in the presence of complement.

"Antibody-dependent cell-mediated cytotoxicity" (ADCC) refers to a cell-mediated reaction in which nonspecific cytotoxic cells that express Fc receptors (FcRs) (e.g. Natural Killer (NK) cells, neutrophils, and macrophages) recognize bound antibody on a target cell and thereby lead to lysis of the target cell.

"Antibody-dependent cell-mediated phagocytosis" (ADCP) refers to a cell-mediated reaction in which phagocytes (such as macrophages) that express Fc receptors (FcRs) recognize bound antibody on a target cell and thereby lead to phagocytosis of the target cell.

CDC, ADCC and ADCP can be measured using assays that are known and available in the art (Clynes et al. (1998) Proc Natl Acad Sci USA 95, 652-6), and as discussed in the Examples. The constant region of an antibody is important in the ability of an antibody to fix complement and mediate cell-dependent cytotoxicity and phagocytosis. Thus, as discussed herein, the isotype of an antibody may be selected on the basis of whether it is desirable for the antibody to mediate cytotoxicity/phagocytosis.

As discussed herein, in an embodiment of the invention, a human IgG2 anti-CD25 antibody that leads to the depletion of Treg cells can be used. For example, a human IgG2 anti-CD25 antibody that elicits a strong CDC response and/or a strong ADCC and/or a strong ADCP response may be used. Methods to increase CDC, ADCC and/or ADCP are known in the art. For example, CDC response may be increased with mutations in the antibody that increase the affinity of C1q binding (Idusogie et al. (2001) J Immunol 166, 2571-5).

ADCC may be increased by methods that eliminate the fucose moiety from the antibody glycan, such as by production of the antibody in a YB2/0 cell line, through the manipulation of the cellular fucose metabolism or antibody glycosylation process, or though the introduction of specific mutations on the Fc portion of human IgG2. ADCP may also be increased by the introduction of specific mutations on the Fc portion of human IgG2

In a particular embodiment of the present invention the antibody is optimised to elicit an ADCC response, that is to say the ADCC response is enhanced, increased or improved relative to other anti-CD25 antibodies, including those that do not inhibit the binding of interleukin 2 to CD25 and, for example unmodified anti-CD25 monoclonal antibodies.

References herein to "does not inhibit the binding of interleukin 2 to CD25" may alternatively be expressed as the anti-CD25 antibody is a non-IL-2 blocking antibody or a "non-blocking" antibody (with respect to the non-blocking of IL-2 binding to CD25 in the presence of the anti-CD25 antibody), i.e. the antibody does not block the binding of interleukin 2 to CD25 and in particular does not inhibit Interleukin-2 signalling in CD25-expressing cells.

References to "does not block", "without blocking" "non-blocking", "non-IL-2-blocking" antibodies and the like (with respect to the non-blocking of IL-2 binding to CD25 in the presence of the anti-CD25 antibody) include embodiments wherein the human IgG2 anti-CD25 antibody of the invention does not block the signalling of IL-2 via CD25. That is the human IgG2 anti-CD25 antibody inhibit less than 50% of IL-2 signalling via CD25 compared to IL-2 signalling in the absence of the antibodies. Preferably the anti-CD25 antibody inhibits less than about 40%, 35%, 30%, preferably less than about 25% of IL-2 signalling compared to IL-2 signalling in the absence of the antibodies. Anti-CD25 non-IL-2 blocking antibodies allow binding to CD25 without interfering with IL-2 binding to CD25, or without substantially interfering with IL-2 binding to CD25. References herein to a non-IL-2 blocking antibody may alternatively be expressed as an anti-CD25 antibody that "does not inhibit the binding of Interleukin-2 to CD25" or as an anti-CD25 antibody that "does not inhibit the signalling of IL-2".

Some anti-CD25 antibodies may allow binding of IL-2 to CD25, but still block signalling via the CD25 receptor. Such anti-CD25 antibodies are not within the scope of the present invention. Instead, the non-IL-2 blocking anti-CD25 antibodies allow binding of IL-2 to CD25 to facilitate at least 50% of the level of signalling via the CD25 receptor compared to the signalling in the absence of the anti-CD25 antibody. Preferably the non-IL-2 blocking anti-CD25 antibody facilitates at least 60%, 65%, 70%, preferably at least 75% of the level of signalling via the CD25 receptor compared to the signalling in the absence of the anti-CD25 antibody. Comparison of IL-2 signalling in the presence and absence of the anti-CD25 antibody agent can occur under the same or substantially the same conditions.

In some embodiments, IL-2 signalling can be determined by measuring by the levels of phosphorylated STAT5 protein in cells, using a standard Stat-5 phosphorylation assay. For example a Stat-5 phosphorylation assay to measure IL-2 signalling may involve culturing PBMC cells in the presence of the anti-CD25 antibody at a concentration of 10ug/ml for 30 mins and then adding varying concentrations of IL-2 (for example 10U/ml or varying concentrations of 0.25U/ml, 0.74U/ml, 2.22U/ml, 6.66U/ml or 20U/ml) for 10 mins. Cells may then be permeabilized and levels of STAT5 protein can then be measured with a fluorescent labelled antibody to a phosphorylated STAT5 peptide analysed by flow cytometry. The percentage blocking of IL-2 signalling can be calculated as follows: % blocking = 100 x [(% Stat5+ cells No Antibody group - % Stat5+ cells 10ug/ml Antibody group) / (% Stat5+ cells No Ab group)].

As used herein, a "chimeric antibody" can refer to an antibody having variable sequences derived from an immunoglobulin from one species, such as rat or mouse antibody, and immunoglobulin constant regions from another species, such as from a human antibody. In some embodiments, the chimeric antibody may have a constant region which is enhanced for inducing ADCC.

**The** antibodies according to the invention may also be partly or wholly synthetic, wherein at least part of the polypeptide chains of the antibodies are synthesized and, possibly, optimized for binding to their cognate antigen. Such antibodies may be chimeric or humanised antibodies and may be fully tetrameric in structure or may be dimeric and comprise only a single heavy and a single light chain.

Antibodies of the present invention may also be monoclonal antibodies. As used herein, "monoclonal antibody" is not limited to antibodies produced through hybridoma technology. **The** term "monoclonal antibody" refers to an antibody that is derived from a single clone, including any eukaryotic, prokaryotic, or phage clone, and not the method by which it is produced.

Antibodies of the present invention may also be human antibodies. As used herein, "human antibody" refers to antibodies having variable regions in which both the framework and CDR regions are derived from human germline immunoglobulin sequences. Furthermore, if the antibody contains a constant region, the constant region also is derived from human germline immunoglobulin sequences. The human antibodies of the invention may include amino acid residues not encoded by human germline immunoglobulin sequences (e.g., mutations introduced by random or site-specific mutagenesis *in vitro* or by somatic mutation *in vivo*).

In a further embodiment, the present invention provides nucleic acid molecules encoding human IgG2 anti-CD25 antibodies as defined herein. The present invention also provides nucleic acid molecules encoding part of the human IgG2 anti-CD25 antibodies as defined herein, for example nucleic acid molecules encoding the heavy chain or light chain variable regions of the anti-CD25 antibodies. In some embodiments, such provided nucleic acid molecules may contain codon-optimized nucleic acid sequences, and/or may be included in expression cassettes within appropriate nucleic acid vectors for the expression in host cells such as, for example, bacterial, yeast, insect, piscine, murine, simian, or human cells. In some embodiments, the present invention provides host cells comprising heterologous nucleic acid molecules (e.g. DNA vectors) that express the desired antibody.

In some embodiments, the present invention provides methods of preparing an isolated human IgG2 anti-CD25 antibody as defined above. In some embodiments, such methods may comprise culturing a host cell that comprises nucleic acids (e.g., heterologous nucleic acids that may comprise and/or be delivered to the host cell via vectors). Preferably, the host cell (and/or the heterologous nucleic acid sequences) is/are arranged and constructed so that the antibody or antigen-binding fragment thereof is secreted from the host cell and isolated from cell culture supernatants

The antibodies of the present invention may be monospecific, bispecific, or multispecific. "Multispecific antibodies" may be specific for different epitopes of one target antigen or polypeptide, or may contain antigen-binding domains specific for more than one target antigen or polypeptide (Kufer et al. (2004) Trends Biotechnol 22, 238-44).

In one aspect of the invention the antibody is a monospecific antibody. As discussed further below, in an alternative aspect the antibody is a bispecific antibody.

As used herein, "bispecific antibody" refers to an antibody having the capacity to bind to two distinct epitopes either on a single antigen or polypeptide, or on two different antigens or polypeptides.

Bispecific antibodies of the present invention as discussed herein can be produced via biological methods, such as somatic hybridization; or genetic methods, such as the expression of a non-native DNA sequence encoding the desired antibody structure in cell line or in an organism; chemical methods (e.g., by chemical coupling, genetic fusion, noncovalent association or otherwise to one or more molecular entities such as another antibody or antibody fragment); or a combination thereof.

The technologies and products that allow producing monospecific or bispecific are known in the art, as extensively reviewed in the literature, also with respect to alternative formats, antibody-drug conjugates, antibody design methods, in vitro screening methods, constant regions, post-translational and chemical modifications, improved feature for triggering cancer cell death such as Fc engineering (Tiller K and Tessier P, 2015 Annu Rev Biomed Eng. 17: 191-216; Speiss C et al., 2015. Molecular Immunology 67 95-106; Weiner G, 2015. Nat Rev Cancer, 15: 361-370; Fan G et al., 2015. J Hematol Oncol 8:130). Such bispecific antibody can be provided in any commercially available format, including Duobody, BiTE DART, CrossMab, Knobs-in-holes, Triomab, or other appropriate molecular format and fragments thereof.

As used herein, "epitope" or "antigenic determinant" refers to a site on an antigen to which an antibody binds. As is well known in the art, epitopes can be formed both from contiguous amino acids (linear epitope) or non-contiguous amino acids juxtaposed by tertiary folding of a protein (conformational epitopes). Epitopes formed from contiguous amino acids are typically retained on exposure to denaturing solvents whereas epitopes formed by tertiary folding are typically lost on treatment with denaturing solvents. An epitope typically includes at least 3, and more usually, at least 5 or 8-10 amino acids in a unique spatial conformation. Methods of determining spatial conformation of epitopes are well known in the art and include, for example, x-ray crystallography and 2-D nuclear magnetic resonance. See, for example, Epitope Mapping Protocols in Methods in Molecular Biology, Vol. 66, Glenn E. Morris, Ed (1996).

In some embodiments, the human IgG2 anti-CD25 antibody can be included in an agent that further comprises a conjugated payload such as a therapeutic or diagnostic agent, in particular for cancer therapy or diagnosis. Anti-CD25 antibody conjugates with radionuclides or toxins may be used. Examples of commonly used radionuclides are, for example, ⁹⁰Y, ¹³¹I, and ⁶⁷Cu, among others, and examples of commonly used toxins are doxorubicin and calicheamicin. In a further embodiment, the anti-CD25 antibody may be modified to have an altered half-life. Methods for achieving an altered half-life are known in the art.

In one embodiment the antibody may block the function of human CD25, preferably in addition to promoting depletion (through ADCC, ADCP and/or CDC) of CD25-expressing cells. Preferably it also blocks the binding of human IL-2 to human CD25, and most preferably blocks human IL-2 signalling in CD25-expressing cells (as discussed above). Alternatively the antibody does not block the binding or signalling of IL-2 to human CD25. Comparison of IL-2 signalling in the presence and absence of the anti-CD25 antibody agent can occur under the same or substantially the same conditions.

In a preferred embodiment of the present invention, the subject of any of the aspects of the invention as described herein, is a mammal, preferably a cat, dog, horse, donkey, sheep, pig, goat, cow, hamster, mouse, rat, rabbit or guinea pig, but most preferably the subject is a human. Thus, in all aspects of the invention as described herein the subject is preferably a human.

As used herein, the terms "cancer", "cancerous", or "malignant" refer to or describe the physiological condition in mammals that is typically characterized by unregulated cell growth.

Examples of cancer include but are not limited to, carcinoma, lymphoma, leukemia, blastoma, and sarcoma. More particular examples of such cancers include squamous cell carcinoma, myeloma, small-cell lung cancer, non-small cell lung cancer, glioma, hepatocellular carcinoma (HCC), hodgkin's lymphoma, non- hodgkin's lymphoma, acute myeloid leukemia (AML), multiple myeloma, gastrointestinal (tract) cancer, renal cancer, ovarian cancer, liver cancer, lymphoblastic leukemia, lymphocytic leukemia, colorectal cancer, endometrial cancer, kidney cancer, prostate cancer, thyroid cancer, melanoma, chondrosarcoma, neuroblastoma, pancreatic cancer, glioblastoma multiforme, cervical cancer, brain cancer, stomach cancer, bladder cancer, hepatoma, breast cancer, colon carcinoma, and head and neck cancer.

In one aspect, the cancer involves a solid tumour. Examples of solid tumours are sarcomas (including cancers arising from transformed cells of mesenchymal origin in tissues such as cancellous bone, cartilage, fat, muscle, vascular, hematopoietic, or fibrous connective tissues), carcinomas (including tumours arising from epithelial cells), mesothelioma, neuroblastoma, retinoblastoma, etc. Cancers involving solid tumours include, without limitations, brain cancer, lung cancer, stomach cancer, duodenal cancer, esophagus cancer, breast cancer, colon and rectal cancer, renal cancer, bladder cancer, kidney cancer, pancreatic cancer, prostate cancer, ovarian cancer, melanoma, mouth cancer, sarcoma, eye cancer, thyroid cancer, urethral cancer, vaginal cancer, neck cancer, lymphoma, and the like.

In one aspect, the cancer involves liquid tumours. Liquid tumours occur in the blood, bone marrow and/or lymph nodes. Cancers that involve liquid tumours including, but not limited, to leukemias, lymphomas, and multiple myelomas, and the like. Such cancers are also known as haematological cancers, and in one aspect the cancer involved is a haematological cancer. Types of leukemia include, but are not limited to, lymphocytic leukemia and myeloid leukemia. Types of lymphoma include, but are not limited to, Hodgkin's lymphoma and non-Hodgkin's lymphoma.

In one aspect the cancers involve CD25 expressing tumours, including but not limited to lymphomas, such as Hodgkin lymphomas, and lymphocytic leukemias, such as chronic lymphocytic leukemia (CLL).

In one aspect of the invention the cancer is selected from melanoma, non-small cell lung cancer, renal cancer, ovarian cancer, bladder cancer, sarcoma and colon cancer. In a preferred aspect of the invention the cancer is selected from melanoma, ovarian, non-small cell lung cancer and renal cancer. In one embodiment, the cancer is not melanoma, ovarian cancer, or breast cancer. In a preferred aspect, the cancer is sarcoma, colon, melanoma or colorectal cancer, or more generally any human cancer for which the 4T1, MCA205, B16, CT26 or MC38 cell line may represent preclinical models for validating compounds as being useful for their therapeutic management.

As used herein, the term "tumour" as it applies to a subject diagnosed with, or suspected of having, a cancer refers to a malignant or potentially malignant neoplasm or tissue mass of any size and includes primary tumours and secondary neoplasms. The terms "cancer", "malignancy", "neoplasm", "tumour", and "carcinoma can be also used interchangeably herein to refer to tumours and tumour cells that exhibit relatively abnormal, uncontrolled, and/or autonomous growth, so that they exhibit an aberrant growth phenotype characterized by a significant loss of control of cell proliferation. In general, cells of interest for detection or treatment include precancerous (e.g., benign), malignant, pre-metastatic, metastatic, and non-metastatic cells. The teachings of the present disclosure may be relevant to any and all cancers.

As used herein, "solid tumours" are an abnormal growth or mass of tissue that usually does not contain cysts or liquid areas, in particular, tumours and/or metastasis (wherever located) other than leukaemia or non-solid lymphatic cancers. Solid tumours may be benign or malignant. Different types of solid tumours are named for the type of cells that form them and/or the tissue or organ in which they are located. Examples of solid tumours are sarcomas (including cancers arising from transformed cells of mesenchymal origin in tissues such as cancellous bone, cartilage, fat, muscle, vascular, hematopoietic, or fibrous connective tissues), carcinomas (including tumours arising from epithelial cells), melanomas, lymphomas, mesothelioma, neuroblastoma, and retinoblastoma.

Particularly preferred cancers in accordance with the present invention include those characterized by the presence of a solid tumour, that is to say the subject does not have a non-solid tumour. In all aspects of the invention as discussed herein, it is preferred that the cancer is a solid tumour, i.e. that the subject has a solid tumour (and does not have a non-solid tumour).

Reference to "treat" or "treating" a cancer as used herein defines the achievement of at least one positive therapeutic effect, such as for example, reduced number of cancer cells, reduced tumour size, reduced rate of cancer cell infiltration into peripheral organs, or reduced rate of tumour metastasis or tumour growth.

Positive therapeutic effects in cancer can be measured in a number of ways (e.g. Weber (2009) J Nucl Med 50, 1S-10S*).* By way of example, with respect to tumour growth inhibition, according to National Cancer Institute (NCI) standards, a T/C ≤ 42% is the minimum level of anti-tumour activity. A T/C < 10% is considered a high anti-tumour activity level, with T/C (%) = Median tumour volume of the treated/Median tumour volume of the control x 100. In some embodiments, the treatment achieved by a therapeutically effective amount is any of progression free survival (PFS), disease free survival (DFS) or overall survival (OS). PFS, also referred to as "Time to Tumour Progression" indicates the length of time during and after treatment that the cancer does not grow and includes the amount of time patients have experienced a complete response or a partial response, as well as the amount of time patients have experienced stable disease. DFS refers to the length of time during and after treatment that the patient remains free of disease. OS refers to a prolongation in life expectancy as compared to naive or untreated individuals or patients.

Reference to "prevention" (or prophylaxis) as used herein refers to delaying or preventing the onset of the symptoms of the cancer. Prevention may be absolute (such that no disease occurs) or may be effective only in some individuals or for a limited amount of time.

In a preferred aspect of the invention the subject has an established tumour, that is the subject already has a tumour, e.g. that is classified as a solid tumour or a liquid tumour. As such, the invention as described herein can be used when the subject already has a tumour, such as a solid tumour or a liquid tumour. As such, the invention provides a therapeutic option that can be used to treat an existing tumour. In one aspect of the invention the subject has an existing solid tumour or a liquid tumour. The invention may be used as a prevention, or preferably as a treatment in subjects who already have a solid tumour or a liquid tumour. In one aspect the invention is not used as a preventative or prophylaxis.

In one aspect, tumour regression may be enhanced, tumour growth may be impaired or reduced, and/or survival time may be enhanced using the invention as described herein, for example compared with other cancer treatments (for example standard-of care treatments for the a given cancer).

In one aspect of the invention the method of treating or preventing cancer as described herein further comprises the step of identifying a subject who has cancer, in particular identifying a subject who has a tumour such as a solid tumour or a liquid tumour. Methods of treatment generally comprise administration of the antibody of the invention, or bispecific antibody of the invention, to the subject in a therapeutically effective amount.

The dosage regimen of a therapy described herein that is effective to treat a cancer patient may vary according to factors such as the disease state, age, and weight of the patient, and the ability of the therapy to elicit an anti-cancer response in the subject. Selection of an appropriate dosage will be within the capability of one skilled in the art. For example 0.01, 0.1, 0.3, 0.5, 1, 2, 3, 4, 5, 6, 7, 8, 9, 10, 20, 30, 40, or 50 mg/kg. In some embodiments, such quantity is a unit dosage amount (or a whole fraction thereof) appropriate for administration in accordance with a dosing regimen that has been determined to correlate with a desired or beneficial outcome when administered to a relevant population (i.e., with a therapeutic dosing regimen).

The antibody according to any aspect of the invention as described herein may be in the form of a pharmaceutical composition which additionally comprises a pharmaceutically acceptable carrier, diluent or excipient. These compositions include, for example, liquid, semi-solid and solid dosage formulations, such as liquid solutions (e.g., injectable and infusible solutions), dispersions or suspensions, tablets, pills, or liposomes. In some embodiments, a preferred form may depend on the intended mode of administration and/or therapeutic application. Pharmaceutical compositions containing the antibody can be administered by any appropriate method known in the art, including, without limitation, oral, mucosal, by-inhalation, topical, buccal, nasal, rectal, or parenteral (e.g. intravenous, infusion, intratumoural, intranodal, subcutaneous, intraperitoneal, intramuscular, intradermal, transdermal, or other kinds of administration involving physical breaching of a tissue of a subject and administration of the pharmaceutical composition through the breach in the tissue). Such a formulation may, for example, be in a form of an injectable or infusible solution that is suitable for intradermal, intratumoural or subcutaneous administration, or for intravenous infusion. The administration may involve intermittent dosing. Alternatively, administration may involve continuous dosing (e.g., perfusion) for at least a selected period of time, simultaneously or between the administration of other compounds.

In some embodiments, the antibody can be prepared with carriers that protect it against rapid release and/or degradation, such as a controlled release formulation, such as implants, transdermal patches, and microencapsulated delivery systems. Biodegradable, biocompatible polymers can be used.

Those skilled in the art will appreciate, for example, that route of delivery (e.g., oral vs intravenous vs subcutaneous vs intratumoural, etc) may impact dose amount and/or required dose amount may impact route of delivery. For example, where particularly high concentrations of an agent within a particular site or location (e.g., within a tumour) are of interest, focused delivery (e.g., in this example, intratumoural delivery) may be desired and/or useful. Other factors to be considered when optimizing routes and/or dosing schedule for a given therapeutic regimen may include, for example, the particular cancer being treated (e.g., type, stage, location, etc.), the clinical condition of a subject (e.g., age, overall health, etc.), the presence or absence of combination therapy, and other factors known to medical practitioners.

The pharmaceutical compositions typically should be sterile and stable under the conditions of manufacture and storage. The composition can be formulated as a solution, microemulsion, dispersion, liposome, or other ordered structure suitable to high drug concentration. Sterile injectable solutions can be prepared by incorporating the antibody in the required amount in an appropriate solvent with one or a combination of ingredients enumerated above, as required, followed by filtered sterilization. Formulations for parenteral administration include, but are not limited to, suspensions, solutions, emulsions in oily or aqueous vehicles, pastes, and implantable sustained-release or biodegradable formulations as discussed herein. Sterile injectable formulations may be prepared using a non-toxic parenterally acceptable diluent or solvent. Each pharmaceutical composition for use in accordance with the present invention may include pharmaceutically acceptable dispersing agents, wetting agents, suspending agents, isotonic agents, coatings, antibacterial and antifungal agents, carriers, excipients, salts, or stabilizers are non-toxic to the subjects at the dosages and concentrations employed. Preferably, such a composition can further comprise a pharmaceutically acceptable carrier or excipient for use in the treatment of cancer that that is compatible with a given method and/or site of administration, for instance for parenteral (e.g. sub-cutaneous, intradermal, or intravenous injection), intratumoral, or peritumoral administration.

While an embodiment of the treatment method or compositions for use according to the present invention may not be effective in achieving a positive therapeutic effect in every subject, it should do so in a using pharmaceutical compositions and dosing regimens that are consistently with good medical practice and statistically significant number of subjects as determined by any statistical test known in the art such as the Student's t-test, the χ²-test, the U-test according to Mann and Whitney, the Kruskal-Wallis test (H-test), Jonckheere-Terpstra test and the Wilcoxon-test.

Where hereinbefore and subsequently a tumour, a tumour disease, a carcinoma or a cancer is mentioned, also metastasis in the original organ or tissue and/or in any other location are implied alternatively or in addition, whatever the location of the tumour and/or metastasis is.

As discussed herein, the present invention relates to depleting CD25+ cells in particular regulatory T cells (Tregs). Thus, in one aspect of the invention, the human IgG2 anti-CD25 antibody depletes or reduces CD25+ cells, in particular tumour-infiltrating regulatory T cells. In one aspect the anti-CD25 antibody of the human IgG₂ isotype depletes or reduces CD25 cells, in particular Tregs and does not inhibit the binding, or at least the signalling, of interleukin 2 to/via the CD25. Alternatively, in one aspect the anti-CD25 antibody of the human IgG₂ isotype depletes or reduces CD25+ cells, in particular Tregs and does inhibit the binding, or at least the signalling, of interleukin 2 to/via the CD25.

In one aspect said depletion is via ADCC. In another aspect, said depletion is via ADCP. The anti-CD25 antibody may also deplete or reduce circulating regulatory T cells. In one aspect said depletion is via ADCC. In another aspect, said depletion is via ADCP.

As such, the invention provides a method for depleting regulatory T cells in a tumour in a subject, comprising administering to said subject an anti-CD25 antibody of the human IgG2 isotype. In one aspect the anti-CD25 antibody of the human IgG2 isotype does not inhibit the binding of interleukin 2 to CD25, or at least the signalling, of IL-2 via CD25, as discussed above.

In a preferred embodiment CD25+ cells are depleted. By "depleted" it is meant that the number, ratio or percentage of CD25+ cells is decreased relative to when an anti-CD25 antibody as described herein, is not administered. In particular embodiments of the invention as described herein, over about 5%, 10%, 20%, 30%, 40%, 50%, 60%, 70%, 80%, 90% or 99% of the CD25+ cells are depleted.

In a particularly preferred embodiment Tregs are depleted in a solid tumour or a liquid tumour. By "depleted" it is meant that the number, ratio or percentage of Tregs is decreased relative to when an anti-CD25 antibody as described herein, is not administered. In particular embodiments of the invention as described herein, over about 5%, 10%, 20%, 30%, 40%, 50%, 60%, 70%, 80%, 90% or 99% of the tumour-infiltrating regulatory T cells are depleted.

Depletion of Tregs cells can be measured using standard methodologies, for example as described in the Examples.

As used herein, "regulatory T cells" ("Treg", "Treg cells", or "Tregs") refer to a lineage of CD4+ T lymphocytes specialized in controlling autoimmunity, allergy and infection. Typically, they regulate the activities of T cell populations, but they can also influence certain innate immune system cell types. Tregs are usually identified by the expression of the biomarkers CD4, CD25 and Foxp3. Naturally occurring Treg cells normally constitute about 5-10% of the peripheral CD4+ T lymphocytes. However, within a tumour microenvironment (i.e. tumour-infiltrating Treg cells), they can make up as much as 20-30% of the total CD4+ T lymphocyte population.

Activated human Treg cells may directly kill target cells such as effector T cells and APCs through perforin- or granzyme B-dependent pathways; cytotoxic T-lymphocyte-associated antigen 4 (CTLA4+) Treg cells induce indoleamine 2,3-dioxygenase (IDO) expression by APCs, and these in turn suppress T-cell activation by reducing tryptophan; Treg cells, may release interleukin-10 (IL-10) and transforming growth factor (TGFβ) *in vivo,* and thus directly inhibit T-cell activation and suppress APC function by inhibiting expression of MHC molecules, CD80, CD86 and IL-12. Treg cells can also suppress immunity by expressing high levels of CTLA4 which can bind to CD80 and CD86 on antigen presenting cells and prevent proper activation of effector T cells.

In a preferred embodiment of the present invention the ratio of effector T cells to regulatory T cells in a solid tumour or a liquid tumour is increased. In some embodiments, the ratio of effector T cells to regulatory T cells in a solid tumour or a liquid tumour is increased to over 5, 10, 15, 20, 40 or 80.

An immune effector cell refers to an immune cell which is involved in the effector phase of an immune response. Exemplary immune cells include a cell of a myeloid or lymphoid origin, e.g., lymphocytes (e.g., B cells and T cells including cytolytic T cells (CTLs)), killer cells, natural killer cells, macrophages, monocytes, eosinophils, neutrophils, polymorphonuclear cells, granulocytes, mast cells, and basophils.

Immune effector cells involved in the effector phase of an immune response express specific Fc receptors and carry out specific immune functions. An effector cell can induce antibody-dependent cell-mediated cytotoxicity (ADCC), e.g., a neutrophil capable of inducing ADCC. For example, monocytes, macrophages, neutrophils, eosinophils, and lymphocytes which express FcαR are involved in specific killing of target cells and presenting antigens to other components of the immune system, or binding to cells that present antigens. An effector cell can also phagocytose a target antigen, target cell, or microorganism. As discussed herein, antibodies according to the present invention may be optimised for ability to induce ADCC.

In some embodiments, a different agent against cancer may be administered in combination with the antibody via the same or different routes of delivery and/or according to different schedules. Alternatively or additionally, in some embodiments, one or more doses of a first active agent is administered substantially simultaneously with, and in some embodiments via a common route and/or as part of a single composition with, one or more other active agents. Those skilled in the art will further appreciate that some embodiments of combination therapies provided in accordance with the present invention achieve synergistic effects; in some such embodiments, dose of one or more agents utilized in the combination may be materially different (e.g., lower) and/or may be delivered by an alternative route, than is standard, preferred, or necessary when that agent is utilized in a different therapeutic regimen (e.g., as monotherapy and/or as part of a different combination therapy).

In some embodiments, where two or more active agents are utilized in accordance with the present invention, such agents can be administered simultaneously or sequentially. In some embodiments, administration of one agent is specifically timed relative to administration of another agent. For example, in some embodiments, a first agent is administered so that a particular effect is observed (or expected to be observed, for example based on population studies showing a correlation between a given dosing regimen and the particular effect of interest). In some embodiments, desired relative dosing regimens for agents administered in combination may be assessed or determined empirically, for example using ex vivo, in vivo and/or in vitro models; in some embodiments, such assessment or empirical determination is made in vivo, in a patient population (e.g., so that a correlation is established), or alternatively in a particular patient of interest.

In another aspect of the invention, a human IgG2 anti-CD25 antibody has improved therapeutic effects when combined with an immune checkpoint inhibitor. A combination therapy with an anti-CD25 antibody of the human IgG2 isotype and an immune checkpoint inhibitor can have synergistic effects in the treatment of established tumours. In one aspect, the combination may lead to enhanced tumour regression, enhanced impairment or reduction of tumour growth, and/or survival time may be enhanced using the invention as described herein, for example compared with either anti-CD25 antibodies or PD-1/PD-L1 blockade alone (directly, using an anti-PD1 antibody, or indirectly, using an anti-PD-L1 antibody). When the anti-CD25 of the human IgG2 isotype of the invention is an anti-CD25 antibody that does not inhibit the binding of IL-2 to CD25, a combination therapy with a human IgG2 anti-CD25 antibody and immune checkpoint inhibitor may also further include the administration of Interleukin-2 at a dosage that is appropriate for the treatment of cancer.

As used herein, "immune checkpoint" or "immune checkpoint protein" refer to proteins belonging to inhibitory pathways in the immune system, in particular for the modulation of T-cell responses. Under normal physiological conditions, immune checkpoints are crucial to preventing autoimmunity, especially during a response to a pathogen. Cancer cells can alter the regulation of the expression of immune checkpoint proteins in order to avoid immune surveillance.

Examples of immune checkpoint proteins include but are not limited to PD-1, CTLA-4, BTLA, KIR, LAG3, TIGIT, CD155, B7H3, B7H4, VISTA and TIM3, and also OX40, GITR, ICOS, 4-1BB and HVEM. Immune checkpoint proteins may also refer to proteins which bind to other immune checkpoint proteins which modulate the immune response in an inhibitory manner. Such proteins include PD-L1, PD-L2, CD80, CD86, HVEM, LLT1, and GAL9.

"Immune checkpoint protein inhibitors" refer to any protein that can interfere with the signalling and/or protein-protein interactions mediated by an immune checkpoint protein. In one aspect of the invention the immune checkpoint protein is PD-1 or PD-L1. In a preferred aspect of the invention as described herein the immune checkpoint inhibitor interferes with PD-1/PD-L1 interactions via anti-PD-1 or anti PD-L1 antibodies.

As such, the present invention also provides a method of treating cancer, comprising administering an anti-CD25 antibody of the human IgG2 isotype and a further therapeutic agent, preferably a checkpoint inhibitor, to a subject. The invention also provides an anti-CD25 antibody of the human IgG2 isotype and a further therapeutic agent, preferably an immune checkpoint inhibitor, for use in the treatment of cancer.

The present invention additionally provides the use of a human IgG2 anti-CD25 antibody and a further therapeutic agent, preferably an immune checkpoint inhibitor, for the manufacture of a medicament for the treatment of cancer. Administration of the human IgG2 anti-CD25 antibody and further therapeutic agent, such as the immune checkpoint inhibitor may be simultaneous, separate or sequential.

The present invention provides a combination of a human IgG2 anti-CD25 and a further therapeutic agent, preferably an immune checkpoint inhibitor, for use in the treatment of cancer in a subject, wherein the human IgG2anti-CD25 antibody and further therapeutic agent, such as the immune checkpoint inhibitor, are administered simultaneously, separately or sequentially. Such a human IgG2 anti-human CD25 antibody can be used specifically in combination with antibodies targeting immune checkpoints that either present or lack sequences that allow ADCC, ADCP, and/or CDC.

In an alternative aspect, the invention provides a human IgG2 anti-CD25 antibody for use in the treatment of cancer, wherein said antibody is for administration in combination with a further therapeutic agent, preferably an immune checkpoint inhibitor. The invention also provides the use of a human IgG2 anti-CD25 in the manufacture of a medicament for treating cancer, wherein said medicament is for administration in combination with a further therapeutic agent, preferably an immune checkpoint inhibitor.

In one embodiment the human IgG2 anti-CD25 antibody used in combination with a further therapeutic agent, preferably an immune check point inhibitor, in the treatment of cancer can be a human IgG2 anti-CD25 antibody that does not inhibit the binding of interleukin 2 to CD25.

The present invention provides a pharmaceutical composition comprising a human IgG2 anti-CD25 antibody, and a further therapeutic agent, preferably an immune checkpoint inhibitor, in a pharmaceutically acceptable medium. As discussed above, the immune checkpoint inhibitor may be an inhibitor of PD-1, i.e. a PD-1 antagonist and/or the human IgG2 anti-CD25 antibody that does not inhibit the binding of interleukin 2 to CD25.

PD-1 (Programmed cell Death protein 1), also known as CD279, is a cell surface receptor expressed on activated T cells and B cells. Interaction with its ligands has been shown to attenuate T-cell responses both *in vitro* and *in vivo.* PD-1 binds two ligands, PD-L1 and PD-L2. PD-1 belongs to the immunoglobulin superfamily. PD-1 signalling requires binding to a PD-1 ligand in close proximity to a peptide antigen presented by major histocompatibility complex (MHC) (Freeman (2008) Proc Natl Acad Sci USA 105, 10275-6). Therefore, proteins, antibodies or small molecules that prevent co-ligation of PD-1 and TCR on the T cell membrane are useful PD-1 antagonists.

In one embodiment, the PD-1 receptor antagonist is an anti-PD-1 antibody, or an antigen binding fragment thereof, which specifically binds to PD-1 and blocks the binding of PD-L1 to PD-1. The anti-PD-1 antibody may be a monoclonal antibody. The anti-PD-1 antibody may be a human or humanised antibody. An anti-PD-1 antibody is an antibody capable of specific binding to the PD-1 receptor. Anti-PD-1 antibodies known in the art include nivolumab and pembrolizumab.

PD-1 antagonists of the present invention also include compounds or agents that either bind to and/or block a ligand of PD-1 to interfere with or inhibit the binding of the ligand to the PD-1 receptor, or bind directly to and block the PD-1 receptor without inducing inhibitory signal transduction through the PD-1 receptor. Alternatively, the PD-1 receptor antagonist can bind directly to the PD-1 receptor without triggering inhibitory signal transduction and also binds to a ligand of the PD-1 receptor to reduce or inhibit the ligand from triggering signal transduction through the PD-1 receptor. By reducing the number and/or amount of ligands that bind to PD-1 receptor and trigger the transduction of an inhibitory signal, fewer cells are attenuated by the negative signal delivered by PD-1 signal transduction and a more robust immune response can be achieved.

In one embodiment, the PD-1 receptor antagonist is an anti-PD-L1 antibody, or an antigen binding fragment thereof, which specifically binds to PD-L1 and blocks the binding of PD-L1 to PD-1. The anti-PD-L1 antibody may be a monoclonal antibody. The anti-PD-L1 antibody may be a human or humanised antibody, such as atezolizumab (MPDL3280A).

The present invention also provides a method of treating cancer, comprising administering a human IgG2 anti-CD25 antibody and an antibody which is an agonist of a T cell activating costimulatory pathway to a subject. Antibody agonists of a T cell activating costimulatory pathway include, without limitation, agonist antibodies against ICOS, GITR, OX40, CD40, LIGHT and 4-1BB.

A further method of treating cancer comprises administering a human IgG2 anti-CD25 antibody and a compound that decreases, blocks, inhibits, and/or antagonizes FcγRIIb (CD32b). Such FcγRIIb antagonist can be a small molecule interfering for FcγRIIb-induced intracellular signalling, modified antibodies that do not engage inhibitory FcγRIIb receptor, or an anti-human FcγRIIb (anti-CD32b antibody. For example, antagonistic anti-human FcγRIIb antibodies have been characterized also for their anti-tumour properties (Roghanian A et al., 2015, Cancer Cell. 27, 473-488; Rozan C et al., 2013, Mol Cancer Ther. 12:1481-91; WO2015173384; WO2008002933).

In a further aspect, the present invention provides a bispecific antibody comprising:
(a) a first antigen binding moiety that binds to CD25; and
(b) a second antigen binding moiety that binds to an immune checkpoint protein, a tumour-associated antigen, an anti-human activatory Fc Receptor antibody (FcgRI, FcgRlla, FcgRIII), or an antagonistic anti-human FcγRIIb antibody;
wherein the bispecific antibody is a human IgG2 bispecific antibody and depletes CD25+ cells, in particular tumour-infiltrating regulatory T cell.

Alternatively, in one aspect the bispecific antibody may comprise a first antigen binding moiety that binds to CD25 and a second antigen binding moiety that binds to an immune checkpoint protein, a tumour-associated antigen, an anti-human activatory Fc Receptor antibody (FcgRI, FcgRlla, FcgRIII), wherein the binding moiety that binds to CD25 is based on a human IgG2 anti-CD25 antibody.

Preferably the human IgG2 bispecific antibody depletes tumour-infiltrating regulatory T cell. In one embodiment, the human IgG2 bispecific antibody binds to at least one activatory Fcγ receptor with high affinity, preferably the activatory Fcγ receptor FcγRIIa. In one embodiment the first antigen binding moiety does not inhibit the binding of interleukin-2 to CD25. Alternatively, the first antigen binding moiety does inhibit the binding of interleukin-2 to CD25.

In a preferred embodiment, the second antigen binding moiety binds to PD-L1.

As used herein, "tumour-associated antigen" refers to antigens expressed on tumour cells, making them distinguishable from non-cancer cells adjacent to them, and include, without limitation, CD20, CD38, PD-L1, EGFR, EGFRV3, CEA, TYRP1 and HER2. Various review articles have been published that describe relevant tumour-associated antigens and the corresponding therapeutically useful antitumor antibody agents (see, for example, Sliwkowski & Mellman (2013) Science 341, 192-8). Such antigens and corresponding antibodies include, without limitation CD22 (blinatumomab), CD20 (rituximab, tositumomab), CD56 (lorvotuzumab), CD66e/CEA (labetuzumab), CD152/CTLA-4 (ipilimumab), CD221/IGF1R (MK-0646), CD326/Epcam (edrecolomab), CD340/HER2 (trastuzumab, pertuzumab), and EGFR (cetuximab, panitumumab).

In one aspect, the bispecific antibody according to the invention as described herein leads to ADCC, or, in one aspect, enhanced ADCC.

The bispecific antibody may bind to a specific epitope on CD25, and a specific epitope on the immune checkpoint protein or tumour-associated antigen as defined herein. In one embodiment the bispecific antibody may bind to a specific epitope on CD25 that does not affect the binding of IL-2 to CD25. In a preferred embodiment, the second antigen binding moiety binds to PD-L1. In a preferred aspect, the present invention provides a bispecific antibody comprising:
(a) a first antigen binding moiety that binds to CD25; and
(b) a second antigen binding moiety that binds to an immune checkpoint protein expressed on a tumour cell
wherein the bispecific antibody is a human IgG2 bispecific antibody and depletes CD25+ cells, in particular tumour-infiltrating regulatory T cells.

In a particular embodiment, the immune checkpoint protein expressed on a tumour cell is PD-L1, VISTA, GAL9, B7H3 or B7H4. Still preferably, the anti-CD25 antibody is a human IgG2 antibody that depletes tumour-infiltrating regulatory T cells. In one particular embodiment, the anti-CD25 antibody is a human IgG2 antibody that binds to at least one activatory Fcγ receptors with high affinity, preferably FcyRlla.

One skilled in the art would be able to produce a bispecific antibody using known methods. The bispecific antibody according to the invention may be used in any of the aspects of the invention as described herein. Preferably, the second antigen binding moiety within the bispecific antibody according to the invention binds to human PD-1, human PD-L1, or human CTLA-4.

In one aspect the bispecific antibody may bind to CD25 and to immune modulatory receptors expressed at high levels on tumour infiltrating Tregs, for example CTLA4, ICOS, GITR, 4-1BB or OX40.

The present invention also provides a kit which comprises a human IgG2 anti-CD25 antibody as described herein, and a further therapeutic agent, preferably an immune checkpoint inhibitor, preferably a PD-1 antagonist (directly, using an anti-PD1 antibody, or indirectly, using an anti-PD-L1 antibody) as discussed herein. In one aspect the immune checkpoint inhibitor is anti-PD-L1. In an alternative embodiment the kit comprises an anti-CD25 antibody as described herein, and an antibody which is an agonist of a T cell activating costimulatory pathway. The kit may comprise instructions for use.

In a further aspect the kit may comprise a human IgG2 anti-CD25 antibody as described herein and a compound that decreases, blocks, inhibits and/or antagonizes FcγRIIb (CD32b), or alternatively and anti-CD25 antibody as described herein and an anti-human activatory Fc Receptor antibody (anti-FcγRI, anti-FcγRIIc or anti-FcγRIIIa).

Any aspect of the invention as described herein may be performed in combination with additional therapeutic agents, in particular additional cancer therapies. In particular, the human IgG2 anti-CD25 antibody and, optionally, the immune checkpoint inhibitor according to the present invention may be administered in combination with co-stimulatory antibodies, chemotherapy and/or radiotherapy (by applying irradiation externally to the body or by administering radio-conjugated compounds), cytokine-based therapy, targeted therapy, monoclonal antibody therapy, a vaccine, or an adjuvant, or any combination thereof.

A chemotherapeutic entity as used herein refers to an entity which is destructive to a cell, that is the entity reduces the viability of the cell. The chemotherapeutic entity may be a cytotoxic drug. A chemotherapeutic agent contemplated includes, without limitation, alkylating agents, anthracyclines, epothilones, nitrosoureas, ethylenimines/methylmelamine, alkyl sulfonates, alkylating agents, antimetabolites, pyrimidine analogs, epipodophylotoxins, enzymes such as L-asparaginase; biological response modifiers such as IFNα, IFN-Y, IL-2, IL-12, G-CSF and GM-CSF; platinum coordination complexes such as cisplatin, oxaliplatin and carboplatin, anthracenediones, substituted urea such as hydroxyurea, methylhydrazine derivatives including N-methylhydrazine (MIH) and procarbazine, adrenocortical suppressants such as mitotane (o,p'-DDD) and aminoglutethimide; hormones and antagonists including adrenocorticosteroid antagonists such as prednisone and equivalents, dexamethasone and aminoglutethimide; progestin such as hydroxyprogesterone caproate, medroxyprogesterone acetate and megestrol acetate; estrogen such as diethylstilbestrol and ethinyl estradiol equivalents; antiestrogen such as tamoxifen; androgens including testosterone propionate and fluoxymesterone/equivalents; antiandrogens such as flutamide, gonadotropin-releasing hormone analogs and leuprolide; and non-steroidal antiandrogens such as flutamide.

The additional cancer therapy may also include the administration of a cancer vaccine. "Cancer vaccines" as used herein refer to therapeutic cancer vaccines administrated to cancer patients and designed to eradicate cancer cells through strengthening patient's own immune responses. Cancer vaccines include tumour cell vaccines (autologous and allogenic), dendritic cell vaccines (*ex vivo* generated and peptide-activated), protein/peptide-based cancer vaccines and genetic vaccines (DNA, RNA and viral based vaccines). Accordingly, therapeutic cancer vaccines, in principle, may be utilized to inhibit further growth of advanced cancers and/or relapsed tumours that are refractory to conventional therapies, such as surgery, radiation therapy and chemotherapy. Tumour cell-based vaccines (autologous and allogeneic) include those genetically modified to secrete soluble immune stimulatory agents such as cytokines (IL-2, IFN-γ, IL12, GMCSF, FLT3L), single chain Fv antibodies against immune modulatory receptors (PD-1, CTLA-4, GITR, ICOS, OX40, 4-1BB) and/or to express on their membrane the ligand for immune-stimulatory receptors such as ICOS-ligand, 4-1BB ligand, GITR-ligand, and/or OX40 ligand amongst others.

The additional cancer therapy may be other antibodies or small molecule reagents that reduce immune regulation in the periphery and within the tumour microenvironment, for example molecules that target TGFbeta pathways, IDO (indoleamine dioxygenase), Arginase, and/or CSF1R.

'In combination' may refer to administration of the additional therapy before, at the same time as or after administration of any aspect according to the present invention.

The invention will now be further described by way of the following Examples, which are meant to serve to assist one of ordinary skill in the art in carrying out the invention and are not intended in any way to limit the scope of the invention, with reference to the drawings in which:

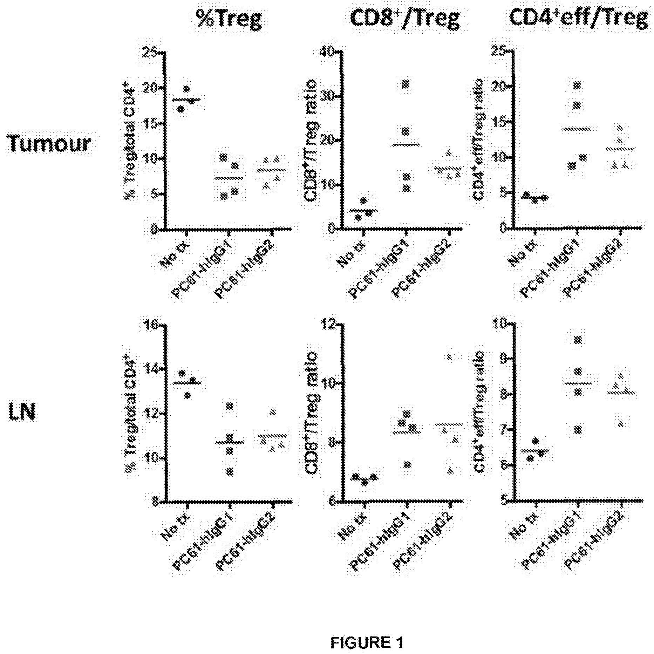

**Figure 1** - shows that anti-CD25 mAb of hIgG1 isotype efficiently depletes Treg cells within tumour and LN, resulting in an increase in the CD4+eff/Treg and CD8+/Treg ratios. The data also show that an anti-CD25 antibody of hIgG2 isotype also promotes efficacious depletion of tumour infiltrating Treg cells and increases the CD4+eff/Treg and CD8+/Treg ratios.

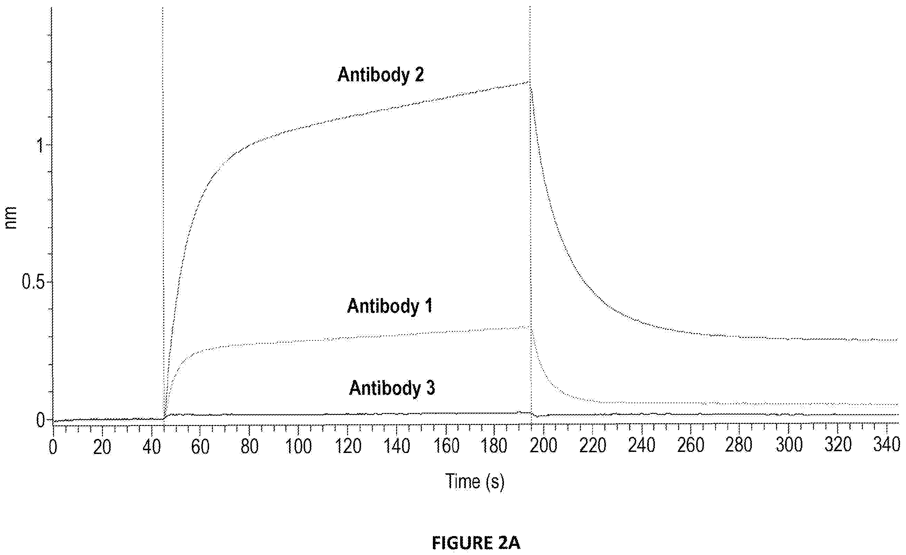

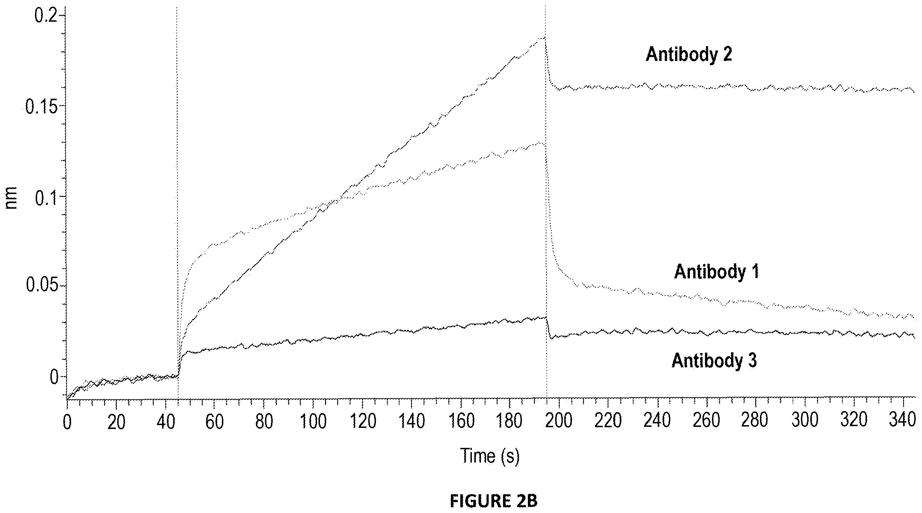

**Figure 2** -Binding of an anti-CD25 non-blocking antibody to Fcγ receptors. Three versions: human IgG1 (Antibody 1), human IgG2 (Antibody 2) and silent human IgG1 (Antibody 3) of the anti-CD25 non-blocking antibody were tested for binding to Fcγ receptors (**A**) CD32a and (**B**) CD32b.

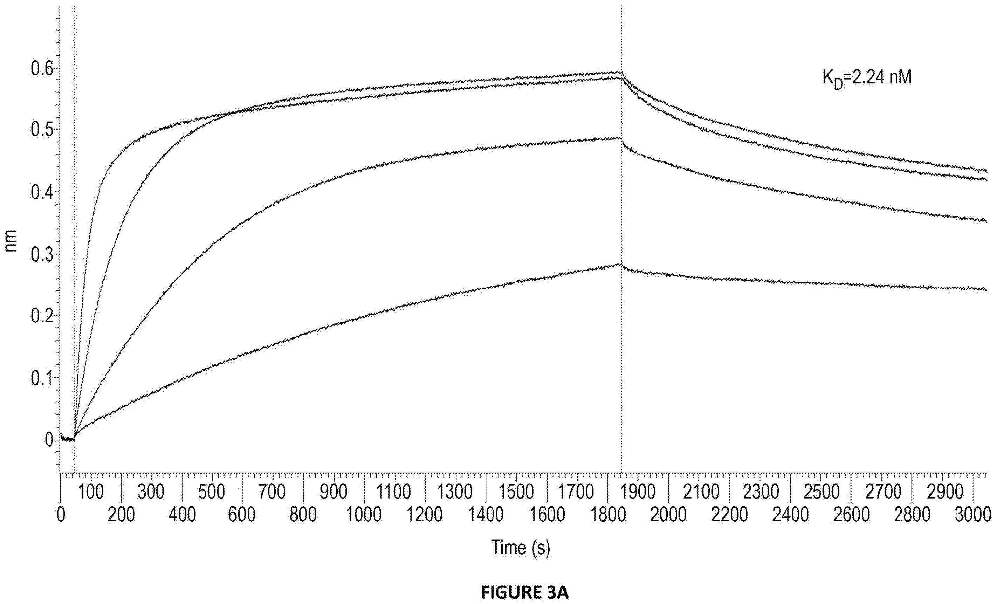

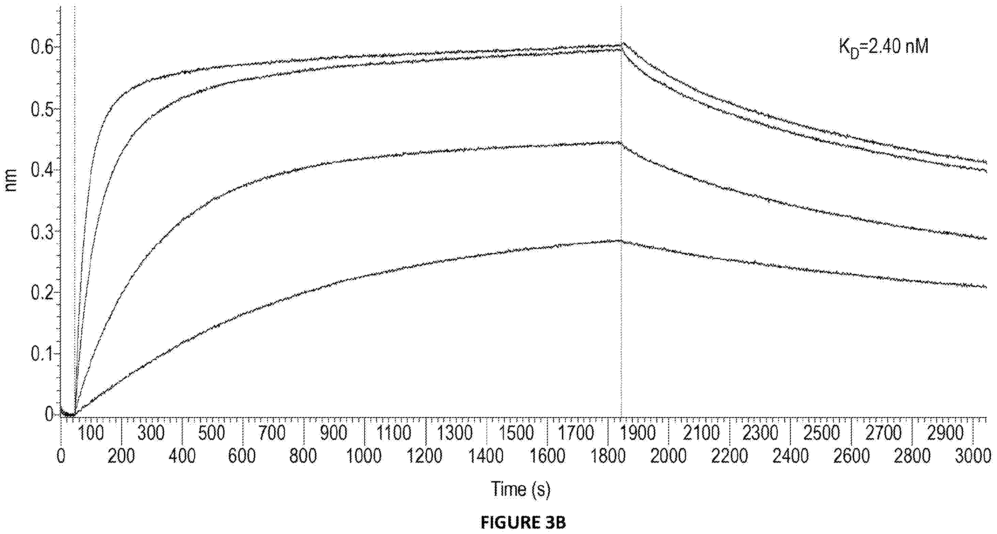

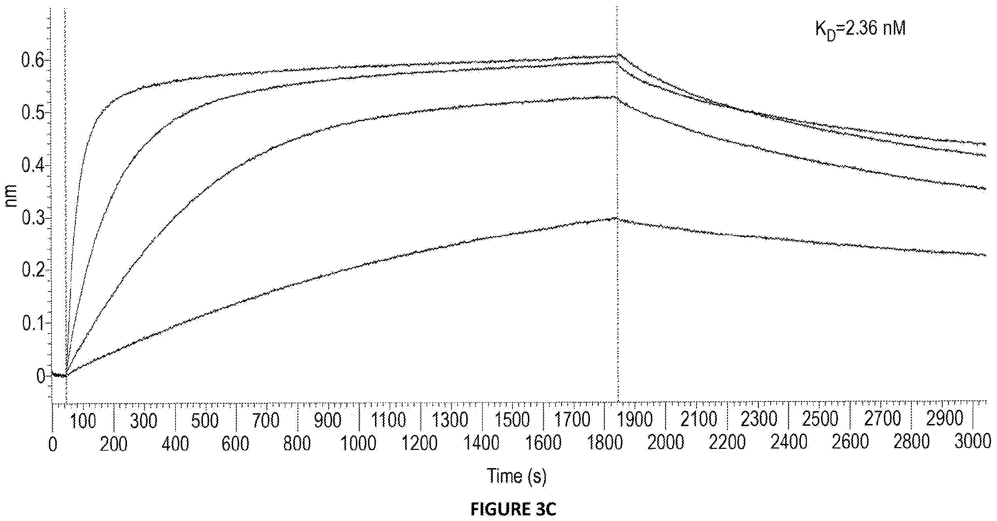

**Figure 3** - KD determinations of three versions of an anti-CD25 non-blocking antibody as **A)** human IgG1 (Antibody 1), **B)** human IgG2 (Antibody 2) and **C)** silent human IgG1 (Antibody 3) to rhCD25-his tag performed by Biolayer interferometry on the Octet Red 96.

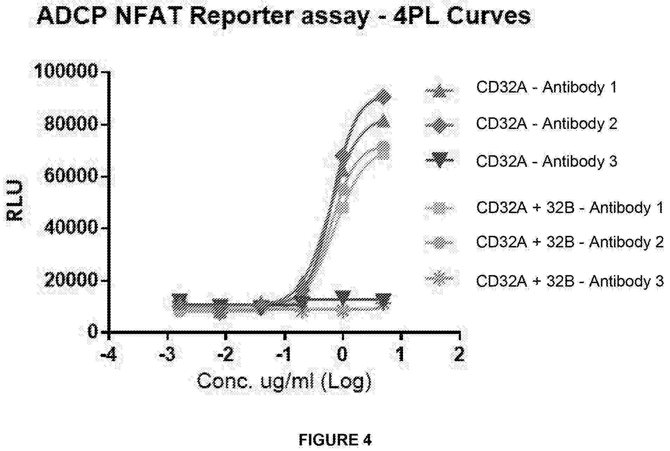

**Figure 4** - Characterisation of human IgG1 (Antibody 1), human IgG2 (Antibody 2) and silent human IgG1 (Antibody 3) with respect to inducing ADCP in a reporter bioassay. CD25 expressing KARPAS 299 cells were co-cultured with Jurkat T cells genetically engineered to express FcγRIIA +/- FcγRIIB and an NFAT-response element that drives luciferase expression (NFAT-RE-luc2) in the presence of varying concentrations of antibodies (as shown in the Figures).

### Examples

### Example 1 - Anti-CD25 of human IgG1 and human igG2 isotypes deplete tumour-infiltrating and peripheral Treg cells in vivo

### Cell lines

MCA205 tumour cells (3-methylcholanthrene-induced weakly immunogenic fibrosarcoma cells; from G. Kroemer, Gustave Roussy Cancer Institute) and 293T cells used for retrovirus production were cultured in Dulbecco's modified Eagle medium (DMEM, Sigma) supplemented with 10% fetal calf serum (FCS, Sigma), 100 U/mL penicillin, 100 µg/mL streptomycin and 2 mM L-glutamine (all from Gibco). K562 cells used for antibody production were cultured in phenol red-free Iscove modified Dulbecco medium (IMDM) supplemented with 10% IgG-depleted FCS (Life Technologies).

### Antibody production

Anti-mouse CD25 (mCD25) monoclonal antibodies (mAbs) of human IgG isotypes were generated using the variable region of anti-mouse CD25 obtained from clone PC61.

The sequence of the variable regions of the heavy and light chains of rat anti-murine CD25 PC61 were resolved from PC-61.5.3 hybridoma (ATCC cat no. TIB-222) by rapid amplification of cDNA ends (RACE) and then inserted in expression vectors that coded for human IgG1 (hIgG1) or human IgG2 (hIgG2) and used for transient transfection of 293T cells for antibody production.

The antibody was purified from supernatants using a protein G HiTrap MabSelect column (GE Healthcare), dialyzed in phosphate-buffered saline (PBS), concentrated and filter-sterilized.

The recloned, anti-mouse CD25 heavy variable chain DNA sequence from PC-61.5.3 antibody encodes for the following protein sequence:

METDTLLLWVLLLWVPGSTGEVQLQQSGAELVRPGTSVKLSCKVSGDTITAYYIHFVKQR
PGQGLEWIGRIDPEDDSTEYAEKFKNKATITANTSSNTAHLKYSRLTSEDTATYFCTTDN
MGATEFVYWGQGTLVTVSS (SEQ ID NO: 3)

The recloned, anti-mouse CD25 light variable chain DNA sequence from PC-61.5.3 antibody encodes for the following protein sequence:

METDTLLLWVLLLWVPGSTGQVVLTQPKSVSASLESTVKLSCKLNSGNIGSYYMHWYQQ
REGRSPTNLIYRDDKRPDGAPDRFSGSIDISSNSAFLTINNVQTEDEAMYFCHSYDGRMY
IFGGGTKLTV (SEQ ID NO: 4)

### In vivo depletion of tumour-infiltrating and peripheral Treg cells

Human Fc-gamma receptor (FcγR) transgenic mice, which express human instead of mouse FcγRs (Smith et al 2012), were challenged with 5 x 10⁵ MCA205 sarcoma cells subcutaneously on day 0. Mice were left untreated (No tx) or treated with 200 µg of anti-mCD25(PC61)-hlgG1 or anti-mCD25(PC61)-hlgG2 on days 5 and 7 after tumour challenge. On day 9, mice were sacrificed and flow cytometry was performed on single cell suspensions from tumours and lymph nodes (LN) in order to quantify the frequency of T cell subpopulations (CD4+Foxp3+ regulatory cells, Treg; CD4+ Foxp3- effector CD4+ T cells, CD4+eff; CD8+ T cells). Results are shown in Figure 1.

In keeping with the ability of human IgG1 mAbs to promote antibody-dependent cell mediated cytotoxicity (ADCC) in vivo, the data demonstrate that the anti-CD25 mAb of hIgG1 isotype efficiently depletes Treg cells within tumour and LN, resulting in an increase in the CD4+eff/Treg and CD8+/Treg ratios. Surprisingly, and contrary to the prevailing wisdom in the field, anti-CD25 of hIgG2 isotype also promoted efficacious depletion of tumour infiltrating Treg cells and also increased the CD4+eff/Treg and CD8+/Treg ratios. The data supports the notion that in vivo, anti-CD25 of a hIgG2 isotype would effectively deplete Treg cells.

### Example 2 - Antibody binding to Fcγ receptors:

Measurement of antibody binding to Fcγ receptors was performed on a Forte Bio Octet Red96 system (Pall Forte Bio Corp., USA) using a standard sequential binding assay. All human recombinant Fcγ receptors were sourced from Sino Biological. All sensors were calibrated off line for 10 minutes in kinetic buffer. A human IgG1 (Antibody 1), a human IgG2 (Antibody 2) and a human IgG1 Fc silent version (Antibody 3), of an anti-CD25 non-IL-2-blocking antibody were tested.

All antibodies had the same variable light chain and variable heavy chain sequences: The constant region of the antibodies provided the non-blocking anti-CD25 antibody with the relevant IgG1, IgG2 or IgG1 Fc silent variant isotype. The IgG1 Fc silent version was engineered as described in Oganesyan V et al, Acta Crystallogr D Biol Crystallogr. 2008 Jun;64(Pt 6):700-704.

For CD16a (FcγRIIIa), 10ug/mL of recombinant His-tagged human CD16 was loaded onto Ni-NTA Biosensors for 1200 seconds. After wash for 15 seconds and a base line step for 45 seconds on kinetic buffer sensors were exposed to 66.6nM of antibody for 150 seconds followed by dissociation in kinetics buffer for 150 seconds.

For CD32b (FcγRIIb), 0.625ug/mL of recombinant His-tagged human CD16 was loaded onto Ni-NTA Biosensors for 900 seconds. After wash for 15 seconds and a base line step for 45 seconds on kinetic buffer sensors were exposed to 66.6 nM of antibody for 150 seconds followed by dissociation in kinetics buffer for 150 seconds.

Data was processed using Forte Bio Data Analysis Software 9.0, with reference subtraction.

The results are shown in Figures 2A-B. The results show that the antibody having a human IgG2 isotype had higher binding affinity to CD32a (FcγRIIa) than the antibody having a human IgG1 isotype. However, both the human IgG1 isotype and human IgG2 isotype antibody had comparable binding affinity to CD32b. The human IgG1 Fc silent variant antibody (used as a negative control), did not bind to CD32a or CD32b.

### Example 3 - Antibody binding to CD25

KD determinations of binding to recombinant human CD25 (from Sino Biological) was performed on a Forte Bio Octet Red96 system (Pall Forte Bio Corp., USA) using a standard sequential binding assay. All sensors were equilibrated off line in kinetic buffer for 10 minutes. 0.625ug/mL of recombinant human CD25 his tagged was loaded onto Ni-NTA Biosensors for 300 seconds. After wash for 15 seconds and a base line step for 45 seconds on kinetic buffer sensors were exposed to a series of antibody concentrations (33.3, 8.3, 2.2 and 0.5 nM) of antibody for 1800 seconds followed by dissociation in kinetics buffer for 1200 seconds.

Data was processed using Forte Bio Data Analysis Software 9.0, with reference subtraction.

The results of the binding of the anti-CD25 antibodies to CD25 are shown in Figures 3A-C. All three anti-CD25 non-blocking antibodies bind to human CD25 with a KD value in the 10⁻⁹M range. Antibody binding to human CD25 was not affected by a change in isotype.

### Example 4 - In vitro ADCP assay

CD25-expressing KARPAS 299 cells, herein called target (T) cells, are incubated for 20 minutes at 37°C with different concentrations of anti-CD25 non-blocking antibodies (human IgG1 (Antibody 1), human IgG2 (Antibody 2) and human IgG1 Fc silent version (Antibody 3)) against CD25 in a low-lgG FBS-supplemented medium (4% FBS in RPMI). ADCP effector (E) cells, are then added to the cell-mAbs mixture at an E:T ratio of 1:1. The effector cells are Jurkat cells stably transfected with a luciferase reporter system and over-expressing CD32A/FcγRIIA +/- CD32B/FcγRIIB. After overnight incubation at 37°C, the cells are lysed and luciferase activity is measured by mean of luminescence release (Relative Luminescence Units (RLU)) from the hydrolysis of a specific luciferase substrate, following manufacturer instruction (Promega Bio-Glo protocol).

As shown in Figure 4 the ability of the antibody having an IgG2 isotype to induce ADCP was comparable with the antibody having a human IgG1 isotype ability to induce ADCP activity. The presence of the inhibitory receptor CD32b only slightly affected the ADCP activity of either the human IgG1 or human IgG2 antibody. The human IgG1 Fc silent variant (used as a negative control), did not induce ADCP in this assay.

The limited impact of the presence of CD32b on ADCP activity, for both the IgG2 and IgG1 antibodies confirms the results shown in Figure 2B, and the comparable weak binding affinity of these antibodies to CD32b.

This data further supports the notion that an anti-CD25 antibody having a human IgG2 isotype would effectively deplete Treg cells.

## Claims

1. An anti-CD25 antibody for use in the treatment of cancer in a human subject, wherein the antibody is a human IgG2 antibody and depletes CD25+ cells and depletes tumor-infiltrating regulatory T-cells.

2. The anti-CD25 antibody for use according to claim 1 wherein the anti-CD25 antibody:
(a) binds to Fcγ receptors with an activatory to inhibitory ratio (A/I) superior to 1; and/or
(b) binds to FcγRIIa with higher affinity than it binds to FcγRIIb.

3. The anti-CD25 antibody for use according to any one of claims 1 to 2, wherein the anti-CD25 antibody has a dissociation constant (Kd) for CD25 of less than 10⁻⁸ M.

4. The anti-CD25 antibody for use according to any one of claims 1 to 3, wherein the antibody does not inhibit the binding of interleukin-2 (IL-2) to CD25.

5. The anti-CD25 antibody for use according to any one of claims 1 to 4, wherein the anti-CD25 antibody is:
- a monoclonal antibody; and/or
- a human antibody.

6. The anti-CD25 antibody for use according to any one of claims 1 to 5, wherein the anti-CD25 antibody elicits an CDC, ADCC and/or ADCP response, preferably an ADCC and/or ADCP response, optionally a monoclonal antibody having ADCP activity.

7. An anti-CD25 antibody for use according to any one of claims 1 to 6 wherein the subject has a solid tumour and/or a liquid tumour.

8. An anti-CD25 antibody for use according to anyone of claims 1 to 7 , wherein the antibody is for administration in combination with a further therapeutic agent.

9. An anti-CD25 antibody for use according to claim 8, wherein the further therapeutic agent is an immune checkpoint inhibitor, optionally a PD-1 antagonist.

10. A combination of an anti-CD25 antibody, as defined in any one of claims 1 to 6, and an immune checkpoint inhibitor for use in the treatment of cancer in a human subject, wherein the anti-CD25 antibody and the immune checkpoint inhibitor are administered simultaneously, separately or sequentially

11. The combination of an anti-CD25 antibody and immune checkpoint inhibitor for use according to claim 10, wherein the subject has a solid tumour and/or a liquid tumour.

12. The combination for use as claimed in claim 10 or 11, wherein the immune checkpoint inhibitor is a PD-1 antagonist, optionally an anti-PD-1 or an anti-PD-L1 antibody.

13. A pharmaceutical composition comprising an anti-CD25 antibody as defined in any one of claims 1 to 6 in a pharmaceutically acceptable medium.

14. A pharmaceutical composition according to claim 13 further comprising a further therapeutic agent.

15. A pharmaceutical composition according to claim 14 wherein the further therapeutic agent is an immune checkpoint inhibitor, optionally wherein the immune checkpoint inhibitor is a PD-1 antagonist, for example an anti-PD-1 antibody or an anti-PD-L1 antibody.

## Patentansprüche

1. Anti-CD25-Antikörper zur Verwendung bei der Behandlung von Krebs bei einem humanen Individuum, wobei der Antikörper ein humaner IgG2-Antikörper ist und CD25+-Zellen depletiert und tumorinfiltrierende regulatorische T-Zellen depletiert.

2. Anti-CD25-Antikörper zur Verwendung nach Anspruch 1, wobei der Anti-CD25-Antikörper:
(a) mit einem Aktivierung-zu-Hemmung-Verhältnis (A/I) über 1 an Fcγ-Rezeptoren bindet; und/oder
(b) mit einer höheren Affinität an FcγRIIa bindet als er an FcγRIIb bindet.

3. Anti-CD25-Antikörper zur Verwendung nach einem der Ansprüche 1 bis 2, wobei der Anti-CD25-Antikörper eine Dissoziationskonstante (Kd) für CD25 von weniger als 10⁻⁸ M aufweist.

4. Anti-CD25-Antikörper zur Verwendung nach einem der Ansprüche 1 bis 3, wobei der Antikörper das Binden von Interleukin-2 (IL-2) an CD25 nicht inhibiert.

5. Anti-CD25-Antikörper zur Verwendung nach einem der Ansprüche 1 bis 4, wobei der Anti-CD25-Antikörper Folgendes ist:
- ein monoklonaler Antikörper; und/oder
- ein humaner Antikörper.

6. Anti-CD25-Antikörper zur Verwendung nach einem der Ansprüche 1 bis 5, wobei der Anti-CD25-Antikörper eine CDC-, ADCC- und/oder ADCP-Antwort, vorzugsweise eine ADCC- und/oder ADCP-Antwort hervorruft, gegebenenfalls ein monoklonaler Antikörper mit ADCP-Aktivität.

7. Anti-CD25-Antikörper zur Verwendung nach einem der Ansprüche 1 bis 6, wobei das Individuum einen soliden Tumor und/oder einen liquiden Tumor aufweist.

8. Anti-CD25-Antikörper zur Verwendung nach einem der Ansprüche 1 bis 7, wobei der Antikörper zur Verabreichung in Kombination mit einem weiteren Therapeutikum vorgesehen ist.

9. Anti-CD25-Antikörper zur Verwendung nach Anspruch 8, wobei das weitere Therapeutikum ein Immuncheckpoint-Inhibitor, gegebenenfalls ein PD-1-Antagonist ist.

10. Kombination aus einem Anti-CD25-Antikörper, wie in einem der Ansprüche 1 bis 6 definiert, und einem Immuncheckpoint-Inhibitor zur Verwendung bei der Behandlung von Krebs bei einem humanen Individuum, wobei der Anti-CD25-Antikörper und der Immuncheckpoint-Inhibitor gleichzeitig, separat oder nacheinander verabreicht werden.

11. Kombination aus einem Anti-CD25-Antikörper und einem Immuncheckpoint-Inhibitor zur Verwendung nach Anspruch 10, wobei das Individuum einen soliden Tumor und/oder einen liquiden Tumor aufweist.

12. Kombination zur Verwendung wie in Anspruch 10 oder 11 beansprucht, wobei der Immuncheckpoint-Inhibitor ein PD-1-Antagonist, gegebenenfalls ein Anti-PD-1- oder ein Anti-PD-L1-Antikörper ist.

13. Pharmazeutische Zusammensetzung, umfassend einen Anti-CD25-Antikörper, wie in einem der Ansprüche 1 bis 6 definiert, in einem pharmazeutisch unbedenklichen Medium.

14. Pharmazeutische Zusammensetzung nach Anspruch 13, ferner umfassend ein weiteres Therapeutikum.

15. Pharmazeutische Zusammensetzung nach Anspruch 14, wobei das weitere Therapeutikum ein Immuncheckpoint-Inhibitor ist, gegebenenfalls wobei der Immuncheckpoint-Inhibitor ein PD-1-Antagonist, zum Beispiel ein Anti-PD-1-Antikörper oder ein Anti-PD-L1-Antikörper, ist.

## Revendications

1. Anticorps anti-CD25 pour une utilisation dans le traitement d'un cancer chez un sujet humain, dans lequel l'anticorps est un anticorps IgG2 humain et déplète les cellules CD25+ et déplète les lymphocytes T régulateurs infiltrant les tumeurs.

2. Anticorps anti-CD25 pour une utilisation selon la revendication 1 dans lequel l'anticorps anti-CD25 :
(a) se lie aux récepteurs Fcγ avec un rapport activation à inhibition (A/I) supérieur à 1 ; et/ou
(b) se lie à FcγRIIa avec une affinité supérieure à celle de sa liaison à FcγRIIb.

3. Anticorps anti-CD25 pour une utilisation selon l'une quelconque des revendications 1 à 2, dans lequel l'anticorps anti-CD25 a une constante de dissociation (Kd) pour CD25 inférieure à 10⁻⁸ M.

4. Anticorps anti-CD25 pour une utilisation selon l'une quelconque des revendications 1 à 3, dans lequel l'anticorps n'inhibe pas la liaison de l'interleukine-2 (IL-2) à CD25.

5. Anticorps anti-CD25 pour une utilisation selon l'une quelconque des revendications 1 à 4, dans lequel l'anticorps anti-CD25 est :
- un anticorps monoclonal ; et/ou
- un anticorps humain.

6. Anticorps anti-CD25 pour une utilisation selon l'une quelconque des revendications 1 à 5, dans lequel l'anticorps anti-CD25 provoque une réponse CDC, ADCC et/ou ADCP, de préférence une réponse ADCC et/ou ADCP, éventuellement un anticorps monoclonal ayant une activité ADCP.

7. Anticorps anti-CD25 pour une utilisation selon l'une quelconque des revendications 1 à 6 dans lequel le sujet a une tumeur solide et/ou une tumeur liquide.

8. Anticorps anti-CD25 pour une utilisation selon l'une quelconque des revendications 1 à 7, dans lequel l'anticorps est destiné à une administration en association avec un agent thérapeutique supplémentaire.

9. Anticorps anti-CD25 pour une utilisation selon la revendication 8, dans lequel l'agent thérapeutique supplémentaire est un inhibiteur de point de contrôle immunitaire, éventuellement un antagoniste de PD-1.

10. Association d'un anticorps anti-CD25, tel que défini dans l'une quelconque des revendications 1 à 6, et d'un inhibiteur de point de contrôle immunitaire pour une utilisation dans le traitement d'un cancer chez un sujet humain, dans laquelle l'anticorps anti-CD25 et l'inhibiteur de point de contrôle immunitaire sont administrés simultanément, séparément ou séquentiellement.

11. Association d'un anticorps anti-CD25 et d'un inhibiteur de point de contrôle immunitaire pour une utilisation selon la revendication 10, dans laquelle le sujet a une tumeur solide et/ou une tumeur liquide.

12. Association pour une utilisation selon la revendication 10 ou 11, dans laquelle l'inhibiteur de point de contrôle immunitaire est un antagoniste de PD-1, éventuellement un anticorps anti-PD-1 ou anti-PD-L1.

13. Composition pharmaceutique comprenant un anticorps anti-CD25 tel que défini dans l'une quelconque des revendications 1 à 6 dans un milieu pharmaceutiquement acceptable.

14. Composition pharmaceutique selon la revendication 13 comprenant en outre un agent thérapeutique supplémentaire.

15. Composition pharmaceutique selon la revendication 14 dans laquelle l'agent thérapeutique supplémentaire est un inhibiteur de point de contrôle immunitaire, éventuellement dans laquelle l'inhibiteur de point de contrôle immunitaire est un antagoniste de PD-1, par exemple un anticorps anti-PD-1 ou un anticorps anti-PD-L1.
